# EUROPEAN PATENT APPLICATION

(11) **EP 4 799 945 A1**
(43) Date of publication of application: **02.09.2026**
(21) Application number: 25160500.2
(22) Date of filing: 27.02.2025
(51) Int. Cl.: B65D 75/08, A61F 13/551, B65D 75/58, B65D 85/07

(54) **ABSORBENT ARTICLE PACKAGES**

(71) Applicant: The Procter & Gamble Company, Cincinnati, OH 45202 (US)
(72) Inventor: REMUS, Michael, 65824 Schwalbach am Taunus (DE); WEISMAN, Paul Thomas, Cincinnati, 45202 (US); BASENDOWSKI, Silke, 65824 Schwalbach am Taunus (DE); MILLAN MALPICA, Arnaldo Rafael, 65824 Schwalbach am Taunus (DE); KRAMKOWSKI, Peter, 65824 Schwalbach am Taunus (DE)
(74) Representative: P&G Patent Germany

(57) **Abstract**

A cuboidal package comprising one or more absorbent articles is disclosed. The package encloses one or more absorbent articles. The package comprises a plurality of panels formed by at least two separate sheets. The second sheet and the first sheet form the top panel and the second sheet is attached to the first sheet such that a hinge is formed, and the top panel is at least partially reclosable via the second sheet. The top panel comprises an opening area.

## Description

### FIELD

The present disclosure is directed to absorbent article packages, more particularly to absorbent article packages comprising a reclosable top panel.

### BACKGROUND

There is an ever pressing need to reduce the environmental impact of products and packaging materials. Accordingly, consumer demand for products made at least partially from renewable resources has increased significantly over the past decade, and has become a driver of innovation for new and improved consumer goods and packaging materials. As such, there is an increased focus on products and packaging materials comprising renewable resources. For example, there is a strong desire in the marketplace for consumer products that comprise natural and bio-sourced materials, recyclable materials, recycled materials, and/or biodegradable materials.

Non-fragile, compressible consumer products such as disposable absorbent articles (e.g., diapers and training pants, disposable adult incontinence pants and feminine hygiene pads) are often packaged and sold at retail in soft packages formed of plastic polymer film. Plastic is preferred as the primary package of consumer goods because plastic may withstand the rigors of a packaging process, given plastic's ability to flex and stretch. Plastic packages, however, tend not to retain their shape, and may collapse upon removal of articles. Packages comprising renewable resources, such as natural fibers (e.g., carton board, cardboard, and paper), may be prone to creasing, cracking, and tearing under packaging processes and opening strain.

The environmental impact can be further reduced by reducing the overall packaging material. On the one hand, disposable absorbent articles are typically sold in multi-packs comprising a plurality of absorbent articles. On the other hand, consumer normally use one disposable absorbent article at a time. Absorbent articles are thus removed from the opened pack multiple times, often at different locations of use. Hence, the remaining articles in the multi-pack are exposed to environmental insults, such as humidity, dust etc., and thus potential contamination after first opening. As a consequence, disposable absorbent articles often are individually packed in addition to the multi-pack packaging. Therefore, there is need for packages providing protection from such environmental insults after first opening. Further, there is still room to improve ease and discretion of retrieval of absorbent articles from packages by consumers.

### SUMMARY

It has surprisingly been found that by providing package materials comprising natural fibers, the packages of the present disclosure are able to retain their shape after opening such that the package can be reclosed via the top panel forming a hinge with the rest of the package. As a consequence, the environmental impact of the package can be reduced due to incorporation of materials from renewable sources and by protecting the articles from contamination after first opening.

A cuboidal package comprising one or more absorbent articles is provided. A first sheet of a first package material comprising natural fibers forms a front panel, a back panel opposite the front panel, a first side panel, a second side panel opposite the first side panel. The package further comprises a top panel, and a bottom panel opposite the top panel. The panels define an interior compartment of the package. One or more absorbent articles are enclosed in the interior compartment. The bottom panel is at least partially formed by the first sheet. The top panel is partially formed by the first sheet. The package further comprises a second sheet of a second package material partially forming the top panel. The second sheet and the first sheet form the top panel. The second sheet is attached the first sheet such that a hinge is formed, and the top panel is at least partially reclosable via the second sheet. The top panel comprises an opening area. Such opening area facilitates the opening, in particular the first opening, of the package.

### BRIEF DESCRIPTION OF THE DRAWINGS

The above-mentioned and other features and advantages of the present disclosure, and the manner of attaining them, will become more apparent and the disclosure itself will be better understood by reference to the following description of example forms of the disclosure taken in conjunction with the accompanying drawings, wherein:
Fig. 1A is a schematic representation of a first sheet;
Fig. 1B is a schematic representation showing the package material sheet of Fig. 1A in a partially folded configuration;
Fig. 1C is a schematic representation of a package with two open ends formed solely by the first sheet;
Fig. 1D is a schematic representation of the package with the top and bottom panel partly formed from the first sheet from the package of Fig. 1C in a partly closed state;
Fig. 2A is a schematic representation showing a bottom panel of a package of the present disclosure, wherein the bottom panel comprises seals in a block bottom configuration;
Fig. 2B is a schematic representation showing three panels including the top panel of a package of the present disclosure with an opening area comprising no adhesive;
Fig. 2C is a schematic representation showing the package of Fig. 2B in open state, except that the opening area is provided with a cover to deactivate the adhesive;
Fig. 2D is a schematic representation showing a top panel of a package of the present disclosure, in which the hinge area and the reclose area are realized by the use of different adhesives applied as glue dots on opposing side edges of the second sheet, while the opening area is realized by deactivated glue dots;
Fig. 3A is a plan view of an example of an absorbent article in the form of a feminine hygiene pad in an unfolded configuration;
Fig. 3B is an edge side view of the feminine hygiene pad of Fig. 3A, shown folded about lateral fold lines in a tri-fold configuration;
Fig. 4A is a plan view of an example of an absorbent article in the form of a disposable diaper, wearer-facing surfaces facing the viewer;
Fig. 4B is a plan view of the diaper of Fig. 4A, shown with side portions folded over and laterally inward about longitudinal side edge fold lines;
Fig. 4C is a plan view of the diaper of Fig. 4B, shown folded about a lateral fold line, wearer-facing surfaces in and outward-facing surfaces out;
Fig. 4D is a side view of the folded diaper shown in Fig. 4C.

### DETAILED DESCRIPTION

The term "absorbent article", as used herein, refers to devices which absorb and contain exudates, and, more specifically, refers to devices which are placed against or in proximity to the body of the wearer to absorb and contain the various exudates discharged from the body. Absorbent articles of the present disclosure include, but are not limited to, diapers, adult incontinence briefs, training pants, diaper holders, menstrual pads, incontinence pads, liners, absorbent inserts, pantiliners, tampons, and the like.

The term "machine direction" or "MD", as used herein, refers to a path that material, such as a package material, follows through a manufacturing process.

The term "cross-machine direction" or "CD", as used herein, refers to a path that is perpendicular to the machine direction in the plane of the material.

The term "natural fibers" as used herein, refers to fibers which comprise cellulose-based fibers, bamboo fibers, and the like. Natural fibers also refers to: nonwoody fibers, such as cotton, abaca, kenaf, sabai grass, flax, esparto grass, straw, jute, hemp, bagasse, milkweed floss fibers, and pineapple leaf fibers; and woody fibers, such as wood or pulp fibers such as those obtained from deciduous and coniferous trees, including softwood fibers, such as northern and southern softwood kraft fibers, hardwood fibers, such as eucalyptus, maple, birch, and aspen. Pulp fibers may be prepared in high-yield or low-yield forms and may be pulped in any known method, including kraft, sulfite, high-yield pulping methods and other known pulping methods. The natural fibers of the present disclosure may be recycled natural fibers, virgin natural fibers or mixes thereof. Additionally, for good mechanical properties in natural fibers, it may be desirable that the natural fibers be relatively undamaged and largely unrefined or only lightly refined. The fibers may have a Canadian Standard Freeness of at least 200, more specifically at least 300, more specifically still at least 400, and most specifically at least 500.

The term "cellulose-based fibers," as used herein, may include regenerated cellulose fiber such rayon or cuprammonium rayon, and high pulping yield fibers, unless specified differently. The term "cellulose-based fibers" also includes chemically treated natural fibers, such as mercerized pulps, chemically stiffened or crosslinked fibers, or sulfonated fibers. Also included are mercerized natural fibers, regenerated natural cellulosic fibers, cellulose produced by microbes, the rayon process, cellulose dissolution and coagulation spinning processes, and other cellulosic material or cellulosic derivatives. Other cellulose-based fibers included are paper broke or recycled fibers and high yield pulp fibers. High yield pulp fibers are those fibers produced by pulping processes providing a yield of about 65% or greater, more specifically about 75% or greater, and still more specifically about 75% to about 95%. Yield is the resulting amount of processed fibers expressed as a percentage of the initial wood mass. Such pulping processes include bleached chemithermomechanical pulp (BCTMP), chemithermomechanical pulp (CTMP), pressure/pressure thermomechanical pulp (PTMP), thermomechanical pulp (TMP), thermomechanical chemical pulp (TMCP), high yield sulfite pulps, and high yield Kraft pulps, all of which leave the resulting fibers with high levels of lignin but are still considered to be natural fibers. High yield fibers are well known for their stiffness in both dry and wet states relative to typical chemically pulped fibers.

### Package Configuration

The package of the present disclosure comprises at least two sheets of packaging material forming a plurality of panels which define an interior compartment and enclose one or more than one absorbent article. When in a closed, sealed respectively, state, such as during transport and on display on a store shelf, the package completely encloses the one or more than one absorbent article. Each of the panels comprises an inner surface - facing inward toward the packaged absorbent article - and an outer surface - facing outward toward the consumer. The outer surface and/or inner surface of one or more panels may comprise inks or dyes which create branding on the package, package information, and/or background color. The branding and/or package information associated with the absorbent articles within the package may be provided on an outer surface of at least one panel. Branding may include logos, trade names, trademarks, icons, and the like, associated with the absorbent articles within the package. Branding may be utilized to inform a consumer of the brand of the absorbent articles within the package. As an example, branding for a package of feminine hygiene pads may comprise the brand name Always^{®}. Package information may include the size of the absorbent articles, the number of absorbent articles within the package, an exemplary image of the absorbent articles contained within the package, recyclability logos, and the like. As an example, package information for a package of feminine hygiene pads may comprise a size indicator, e.g., "Size 1."

The package materials of the present disclosure may be supplied by a manufacturer of package materials to an absorbent article manufacturer. The package materials may be pre-formed to some extent into a finished package shape, or the manufacturer of package materials may simply provide rolls of the package materials to the absorbent article manufacturer. Preferably, the first package material comprising natural fibers may be provided as a continuous material web. The continuous material web of first package material may be separated into first sheets.

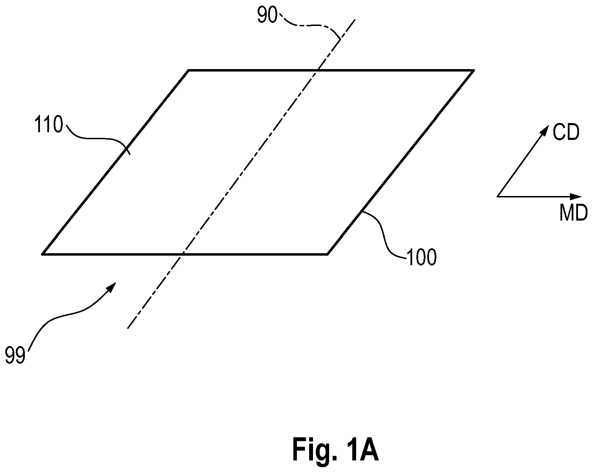

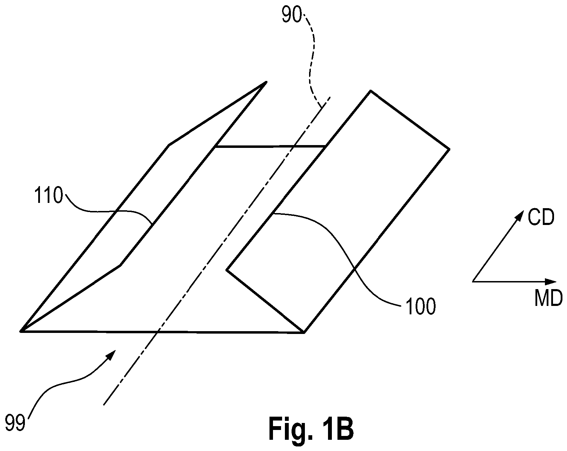

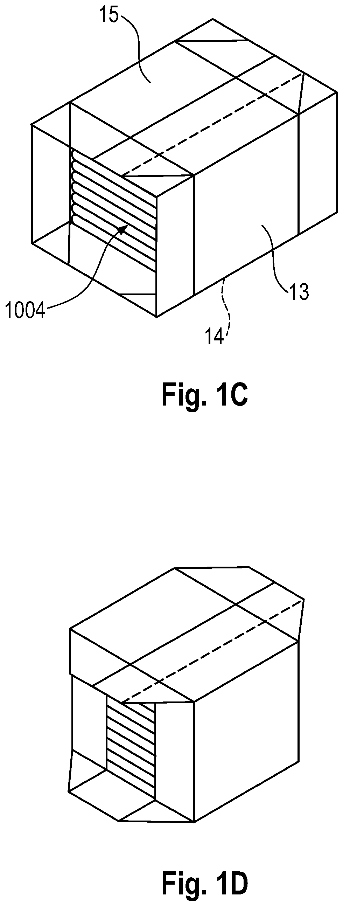

The first sheet 99 may be folded and erected to form the front panel 14, the back panel 15, the first side panel 12, and the second side panel 13. In particular, a sleeve may be formed from the first sheet of first package material. The sleeve may preferably be formed by sealing the first sheet 99 to form a single linear seam. Edge portions 100, 110 of the first sheet 99 may be folded towards each other and subsequently sealed to form a seam. For example, side edge portions 100, 110 of the first sheet 99 may be brought inward towards a longitudinal centerline of the first sheet to form a hoop seam 95 (see Fig. 1C). These edge portions may be overlapped with one another and sealed together to form an overlap seam. Alternatively, the edge portions 100 and 110 may be joined together on their respective inner surfaces to form a butt seam. Butt seams tend to not lay as flat as an overlap seam and are thus less preferred. The linear seam may be substantially perpendicular to the cross-machine direction or preferably substantially perpendicular to the machine direction. The seam line may be comprised by one panel selected from the group consisting of the front panel 14, the back panel 15, the first side panel 12, and the second side panel 13; or may correspond to an edge, a fold line respectively, selected from the group consisting of the edge connecting the front panel 14 and the first side panel 12, the edge connecting the front panel 14 and the second side panel 13, the edge connecting the back panel 15 and the first side panel 12 or the edge connecting the back panel 15 and the second side panel 13.

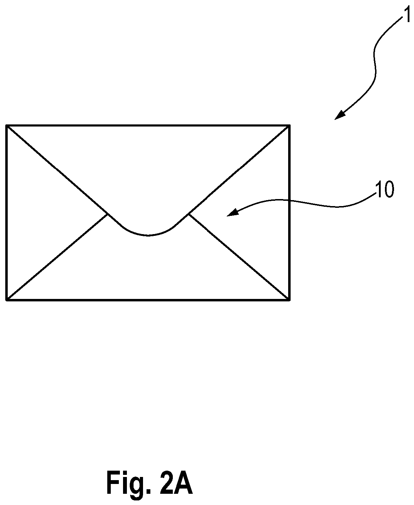

The one or more absorbent articles 1004 may be inserted in the sleeve via an open side of the sleeve. Subsequently, the top panel 11 may be partially and the bottom panel 10 may be at least partially formed by the first sheet 99 of the first package material, preferably by means of an envelope fold. In one embodiment, the bottom panel 10 may be completely formed by envelope folding the first sheet 99. Such a so-called block-bottom configuration is shown in Fig. 2A. Hence, one open end, corresponding to the bottom part of the package, of the sleeve may be sealed by means of an envelope fold.

The front panel 14 and the back panel 15 each have a height H_{P} and a width W_{P} corresponding to the width and height of the package. The first side panel 12 and the second side panel 13 each have a height H_{P} and a width D_{P} corresponding to the corresponding to the depth and height of the package. The top panel 11 and bottom panel 10 each have a length D_{P} and a width W_{P} corresponding to the depth and width of the package. In one example, H_{P} and D_{P} may substantially the same. Further, W_{P} may be larger than H_{P} and/ or D_{P}.

The top panel 11 is partially formed by the first sheet 99of first package material, preferably by means of an envelope fold of the sleeve, and partially formed of the second sheet of the second package material. Preferably, the second package material comprises natural fibers. As such, the part of the top panel 11 formed by the first sheet 99 defines an opening 71, which is covered by the second sheet. Preferably, the second sheet may be attached to the parts of the top panel 11 formed by the first sheet 99 to enclose the one or more absorbent articles 1004. The part of the top panel 11 formed by the first sheet 99 may be partially folded over itself to form a folded area. At least a portion of the folded area may be not adhesively attached to itself. The second sheet may at least partly cover the folded area and the folded area may be able to unfold upon removal of the second sheet from the folded area.

The opening 71 defined by the part of the top panel 11 formed by the first sheet 99 may have a length L_{O} and a width W_{O}. W_{O} is parallel to W_{P} and L_{O} is parallel to D_{P}. The length L_{O} may be parallel to the machine direction, while the width Wo may be parallel to the cross-machine direction. L_{O} may be less than the dimension of the enclosed absorbent articles in the direction parallel to L_{O} and/ or W_{O} may be less than the dimension of the one or more enclosed absorbent articles in the direction parallel to W_{O}. Each absorbent article may be folded at least once and arranged within the package such that a fold nose faces the top panel 11. This facilitates disposal of the articles through the opening. If folded, the one or more enclosed absorbent article will have a folded width FW measured as the distance between side edges, a folded height FH measured as the distance between fold nose 30 and the end edges (in the case of a bi-fold configuration) or between the two fold noses 30 (in the case of a tri-fold configuration), and a side width (SW). The folded width (FW) forms the flat, broad face of the folded absorbent article, while the side width (SW) forms the narrow folded side of the absorbent article. The average folded width FW is calculated by the dividing the sum of the FWs of all enclosed articles by the total number of articles. In one particular example, L_{O} may be less than the average folded width FW of the enclosed absorbent articles and/ or W_{O} may be less than the average folded width FW of the one or more enclosed absorbent articles. Upon at least partial unfolding of the folded area, the opening 71 may expanded such that the absorbent articles fit through the expanded opening. Due to such configuration, retrieval of the absorbent articles from the package is facilitated. In particular for folded articles, the fold nose is rigid enough to partly unfold the folded area upon removal.

In one example, the bottom panel 10 is partially formed by the first sheet 99 of first package material, preferably by means of an envelope fold of the sleeve, and partially formed of a third sheet of a third package material comprising natural fibers. Preferably, the third sheet may be attached to the parts of the bottom panel 10 formed by the first sheet 99 to enclose the one or more absorbent articles 1004. In other word, a sleeve may be formed from a first sheet 99 forming four panels of the package, which may be envelope folded to partially form the top 11 and bottom panel 10 after the one or more absorbent articles 1004 were inserted. The package 1 may then be sealed by attaching a second and third sheet of package material to the first sheet 99 to form the top 11 and bottom panel 10. Thus, the open ends of the sleeve may partially be closed via folding over the first sleeve and then sealed via attaching separate, sticker-like sheets of packaging material.

The second sheet may be substantially rectangular. The third sheet may be substantially rectangular. The second and the third sheet may have substantially the same dimensions. The second sheet has a length L_{S} and a width W_{S} wherein W_{S} is parallel to W_{P} and L_{S} is parallel to D_{P}. W_{S} may be shorter than W_{P}. W_{S} may be from 70% to 99.5%, preferably from 80% to 99%, more preferably from 85% to 98%, even more preferably from 90% to 97% or even from 92% to 96%of W_{P}. By such a relation, it can be ensured that there is sufficient overlap between the parts of the top panel 11 formed by the first and the second sheet, while the second sheet does not exceed over the edge of the top panel even within typical manufacturing variations. Accordingly, L_{S} may be shorter than D_{P}. L_{S} may be from 70% to 99.5%, preferably from 80% to 99%, more preferably from 85% to 98%, even more preferably from 90% to 97% or even from 92% to 96%of D_{P}.

The second sheet may comprise a grip tab extending from one side edge of the second sheet, preferably from the side edge opposite to the hinge formed by the second sheet and the first sheet 99. The grip tab may be integral with or attached to the second sheet. The grip tab may be comprised by the opening area 199 or neighboring the opening area 199. When the grip tab is integral with the second sheet, the grip tab may be comprised by the opening area 199. By this, opening of the package may be facilitated.

### Top and bottom panel configuration

The second sheet and the first sheet 99 form the top panel 11 and the second sheet is attached to the first sheet such that a hinge is formed. The hinge line may be formed on the boarder of or within the area where the first and second sheet are attached to each other or the second sheet may act as a lever and the hinge line may be correspond to the edge of the top panel 11 formed by the first sheet or be located within the area of the top panel formed by the first sheet. The top panel 11 is at least partially reclosable via the second sheet.

The top panel 11 comprises an opening area 199. The opening area 199 is provided to facilitate opening of the cuboidal package 1. This is achieved, by the second sheet being not or only lightly attached to the first sheet 99 within the opening area 199. In the opening area 199, the second sheet may not be attached to the first sheet 99 or may be attached to the first sheet 99 such that the opening area 199 exhibits a Peak Peel Force at first peel of from 0.0 N to 10 N, preferably from 0.0 N to 5.0 N. In the opening area 199, the second sheet may not be attached to the first sheet 99 or may be attached to the first sheet 99 such that second sheet may be lifted with only minimal force. The opening area 199 may be provided such that a consumer can push a fingertip between the first 99 and second sheet in the opening area 199.

In one example, the second sheet is attached to the first sheet of first package material via one or more adhesives. A surface area of the second sheet covering the opening 71 defined by the part of the top panel 11 formed by the first sheet 99 and facing the enclosed absorbent articles 1004 may be free of adhesive. Hence, the enclosed absorbent articles are not in contact and thus potentially contaminated with adhesive.

The type of adhesives utilized for the seams and for attaching the second and/ or optional third sheet to the first sheet to form the packages of the present disclosure may impact the recyclability of the package as well. As an example, adhesives that can dissolve in water during the re-pulping step or the disintegration step of the paper recycling process may be particularly suitable for the packages of the present disclosure. Such adhesives include starch based adhesives, polyvinyl acetate based adhesives, and polyethylene oxide based adhesives. A suitable example of a starch based adhesive is available from LD Davis located in Monroe, North Carolina, under the trade name AP0420CR. A suitable example of a polyvinyl acetate based adhesive is available from Sekisui Chemical Company, located in Osaka, Japan, under the trade name Selvol 205. A suitable example of a polyethylene oxide based adhesive is available from Dow Chemicals Co. located in Midland, Michigan, under the trade name WSR N-80.

Water-dispersible adhesives may similarly be utilized. Suitable examples of water dispersible adhesives include thermoplastic elastomer based adhesives and polyvinyl acetate based adhesives. A suitable example of a thermoplastic elastomer based adhesive is available from Actega located in Blue Ash, Ohio, under the trade name Yunico 491. A suitable example of a polyvinyl acetate based adhesive is available from Bostik located in Milwaukee, Wisconsin, under the trade name Aquagrip 4419U01. Another suitable example of a polyvinyl acetate based adhesive is available from HB Fuller under the trade name PD-0330.

Without wishing to be bound by theory, it is believed that packages of the present disclosure which utilize adhesives dissolvable in water may comprise a higher weight percentage of such adhesives than adhesive which are only water dispersible. For example, packages comprising water dissolvable adhesives may comprise a first weight percentage of adhesive while packages comprising water dispersible adhesives may comprise a second weight percentage of adhesive. The first weight percentage may be greater than the second weight percentage for the purposes of recycling the package material.

In one example, an area, preferably an area comprising at least one corner or at least partially at least one side edge, of the second sheet may be permanently attached to the first sheet to form a hinge area. Permanently attached in this context means that the first and second sheet cannot be separated over the whole hinge area without damaging, e.g. tearing, the first or second package material. The hinge area may be substantially rectangular.

The hinge area may be rectangular and may at least partially comprise one side edge of the second sheet which is parallel to the width of the package W_{P} or one side edge of the second sheet which is parallel to the depth of the package D_{P}. The rectangular hinge area may have a length L_{H} and a width W_{H}. W_{H} may be parallel to W_{P} and L_{H} is parallel to D_{P}. In this context, the hinge area may have a maximum width W_{Hmax} extending from the side edge of the opening, which is defined by the part of the top panel 11 formed by the first sheet, to the side edge of the second sheet in the direction parallel to the width of the package W_{P} and maximum length L_{Hmax} corresponding to L_{S} or alternatively have maximum length L_{Hmax} extending from the side edge of the opening, which is defined by the part of the top panel 11 formed by the first sheet, to the side edge of the second sheet in the direction parallel to the depth of the package D_{P} and maximum width W_{Hmax} corresponding to W_{S}. In other words, the hinge area may have a maximum width W_{Hmax} = (W_{P}-W_{O})/2 and maximum length L_{Hmax} = L_{S} or alternatively may have a maximum length L_{Hmax} = (D_{P}-L_{O})/2 and maximum width W_{Hmax} = W_{S}.

The hinge area may be rectangular and may comprise one corner of the second sheet. In this context, the hinge area may preferably have a maximum width W_{Hmax} extending from the side edge of the opening 71, which is defined by the part of the top panel 11 formed by the first sheet, to the side edge of the second sheet in the direction parallel to the width of the package W_{P} and a maximum length L_{Hmax} extending from the side edge of the opening 71 to the side edge of the second sheet in the direction parallel to the depth of the package D_{P}. In other words, the hinge area may have a maximum width W_{Hmax} = (W_{P}-W_{O})/2 and a maximum length L_{Hmax} = (D_{P}-L_{O})/2.

In one example, an area, preferably an area comprising at least partially at least one side edge or at least one corner, of the second sheet may be reclosably attached to the first sheet to form a reclose area. Reclosably attached means that the first and second sheet can be separated in the reclose area without substantially damaging the first and second package material and that the second sheet can be re-attached to the first sheet. Such reclosability may be achieved via adhesively attaching the second sheet to the first sheet in the reclose area, but could also be achieved mechanically, e.g. by interlocking between the second and first sheet. In an alternative example, the second sheet may be attached to first sheet only prior to opening and then reclose the package without adhering to the first sheet due to an imparted tendency to return to the closed position. Such a tendency can be realized by employing a higher basis weight material as second sheet as described below.

The opening area 199 may comprise a corner of the second sheet, a perimeter position of the second sheet, or combinations thereof. In particular, the opening area 199 may comprise a corner of the second sheet and at least partially comprise the neighbouring side edges of the second sheet.

The opening area 199 may preferably at least partially comprise one side edge of the second sheet, the hinge area may preferably at least partially comprise one side edge of the second sheet and the reclose area may at least partially comprise two side edges of the second sheet. Alternatively, the opening area 199 may preferably at least partially comprise one corner and partially two side edges of the second sheet, the hinge area may comprise one corner and partially two side edges of the second sheet and the reclose area may comprise two corners and at least partially comprise four side edges of the second sheet. The hinge area and the opening area 199 may be at least partially located on opposing side edges and/ or opposing corners of the second sheet. In particular, the hinge area and the opening area 199 may be at least partially located on opposing corners of the second sheet and the perimeter of the second sheet, which are not comprised by the opening and hinge area, may be comprised by the reclose area. By such configuration, it can be ensured that the second sheet stays close to the part of the first sheet forming the top panel also in the opening area 199, such that the articles are enclosed in the package prior to first opening. The reclose area may at least partially comprise one side edge of the second sheet which is parallel to the width of the package W_{P} or one side edge of the second sheet which is parallel to the depth of the package D_{P}.

In one example, the hinge area may at least partially comprise one side edge of the second sheet which is parallel to the depth of the package D_{P} and the reclose area may at least partially comprise the opposing side edge of the second sheet.

In one example, the hinge area may comprise one corner of the second sheet of the package and the opening area 199 may comprise the opposing corner of the second sheet. The opening area 199 may comprise one corner of the second sheet of the package, the hinge area may comprise one corner of the second sheet of the package and the reclose area may comprise the other two corners of the second sheet.

The opening area 199 may comprise a total length of the edge of the second sheet of from 0.4 cm to 4.0 cm, preferably from 0.5 cm to 3.5 cm, more preferably from 0.6 cm to 3.0 cm, more preferably from 0.7 cm to 2.5 cm, even more preferably from 0.8 cm to 2.0 cm or even from 0.9 to 1.5 cm. Such length may be measured from as distance between the reclose area, glue dots respectively, along the perimetric edge of the second sheet. Such length may balance the ease of opening with the opening being sufficiently small to not open during transport and protecting the enclosed articles from the environment during transport and on the shelf. Although the length is measured along the edge of the second sheet, the opening area 199 may be realized by de-activating adhesive or glue or providing an anti-adhering coating on the first sheet forming part of the top panel.

The opening area 199 may comprise a total area of the second sheet of from 0.2 cm² to 10.0 cm², preferably from 0.5 cm² to 8.0 cm², more preferably from 1.0 cm² to 6.0 cm², even more preferably from 1.5 cm² to 5.0 cm² or even from 2.0 cm² to 4.0 cm². Such area may be measured as the area between the perimetric edge of the second sheet and the edge of the first sheet 99 parallel to the edge of the second sheet comprised by the top panel. Such area may balance the ease of opening with the opening being sufficiently small to not open during transport and protecting the enclosed articles from the environment during transport and on the shelf.

The opening area 199 may be either free of adhesives or the adhesive in the opening area 199 may be covered by a coating or cover material. In other words, the opening area 199 may be free of adhesive or the adhesive in the opening area 199 may be deactivated by means of a coating or a cover material. Such cover coatings may exemplarily be polyolefin-based coatings, in particular polyethylene coatings, or siliconized coatings. A cover material may exemplarily be a tape material, a polymeric film material or a paper-based material. In particular, the first and second sheet in the opening area 199 may be free of adhesive. Alternatively, the adhesive on the second sheet in the opening area 199 may be covered by a coating or cover material. A non-adhesive side of the cover material in such configuration should be facing the first sheet 99. Alternatively, the adhesive on the first sheet 99 in the opening area 199 may be covered by a coating or cover material. A non-adhesive side of the cover material in such configuration should be facing the second sheet.

In the reclose area, the surface of the second sheet facing the first sheet may comprise an adhesion-reducing coating and the surface of the first sheet facing the second sheet may comprise an adhesive. Alternatively, the surface of the second sheet facing the first sheet may comprise an adhesive and the surface of the first sheet facing the second sheet comprises an adhesion-reducing coating. Adhesion-reducing coatings are known to the skilled person. Adhesion-reducing coatings may exemplarily be polyolefin-based coatings, in particular polyethylene coatings, or siliconized coatings. By such coating, both closability for multiple opening-reclosing cycles and discretion due to reduced opening sounds can be enabled.

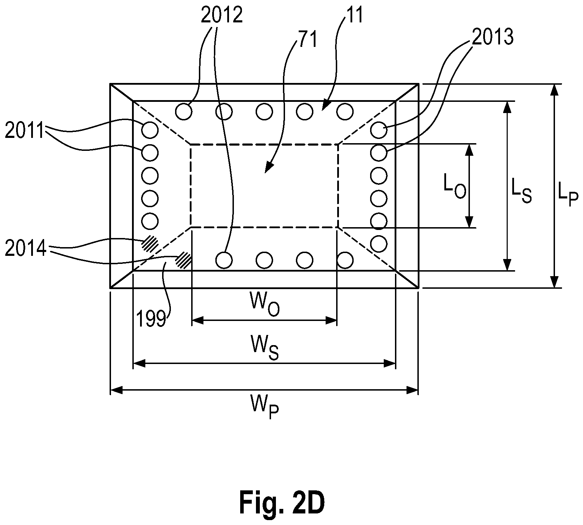

In one example, the hinge area may comprise a different adhesive than the reclose area. In particular, the hinge area may comprise an adhesive with increased adhesion in comparison with the adhesive comprised by the reclose area. Alternatively, the hinge area and reclose area may comprise the same adhesive and the reclose area comprises an adhesion-reducing coating. In one example, three different adhesives may be applied. The hinge area may comprise a third adhesive 2013 in the area formed by the first sheet 99 along one side edge, and the reclose area may comprise a first adhesive 2011 in the area formed by the first sheet 99 along the side edge opposing the hinge area and a second adhesive 2012 in the area formed by the first sheet 99 along the two side edge bridging the side edge comprising the first adhesive 2011 and the side edge comprising third adhesive 2013. In the opening area 199, dots of deactivated adhesive 2014, e.g. by a coeating, may be present. This is exemplarily shown in Fig. 2D. The first adhesive 2011 may be re-adhering after first opening. The second adhesive 2012 may be non-adhering after first opening. The third adhesive 2013 may be permanently adhering. In the opening area The opening area 199 may be provided such that a consumer can push a fingertip between the first 99 and second sheet in the opening area 199., either no adhesive may be present or the first and/ or second adhesive 2011, 2012 may covered by a coating or cover material.

The respective adhesive may cover the whole reclose area and/ or hinge area. In one example, the respective adhesive may be applied non-continuously in the reclose area and/ or hinge area. In particular, the adhesive may be applied in stripes and/ or as dots 201, in particular lines of dots 201 in the reclose area and/ or the hinge area.

The adhesion in the opening area 199 may be provided such that a consumer can push a fingertip between the first 99 and second sheet in the opening area 199., the hinge area and reclose area may be characterized via the Peak Peel Force.

For example, the hinge area may exhibit a Peak Peel Force of at least 10 N, preferably at least 20 N according to the Seal Strength Test Method disclosed herein. Such a Peak Peel Force ensures that the hinge area can withstand any pull forces during use of the packages by the consumer.

The reclose area may exhibit a Peak Peel Force at first peel of from 0.1 N to 20 N, preferably from 0.5 N to 19 N, more preferably from 1.0 N to 18 N, even more preferably from 2.0 N to 17 N or even from 3.0 N to 15 N according to the Seal Strength Test Method disclosed herein. Due to such a Peak Peel Force at first peel, it is ensured that the package stays in closed state during transport while opening via peeling prior to first use is facilitated. The reclose area may exhibit a Peak Peel Force at fifth peel of 0.1 N to 18 N, preferably from 0.5 N to 17 N, more preferably from 1.0 N to 16 N, even more preferably from 2.0 N to 15 N or even from 3.0 N to 13 N according to the Seal Strength Test Method disclosed herein.

In particular, the Peak Peel Force at first peel of the reclose area may be less than the Peak Peel Force of the hinge area according to the Seal Strength Test Method disclosed herein.

The package may comprise an opening indicium. The opening indicium may guide the consumer on where and potentially how to open and reclose the package of the present disclosure. The opening indicium may comprise text, arrows and similar indicators. Preferably, the opening indicium may be comprised by an outer surface of the opening area The opening area 199 may be provided such that a consumer can push a fingertip between the first 99 and second sheet in the opening area 199. Alternatively or additionally, the indicium may be comprised by an outer surface of the grip tab.

### Package Materials

The first and the second package material may be the same or different. The optional third package material may be the same or different from the first and/ or second package material. In one embodiment, the second and third package material may be the same. In one embodiment, first and third package material may be the same, while the second package material is different from the second and third package material. For example, the second package material may be chosen to be at least partially translucent. Alternatively, the second package material may comprise a translucent window. The translucent window may correspond to the opening 71 defined by the part of the top panel 11 formed by the first sheet. Thus, the window may have the same length L_{O} and width W_{O} as the opening. By this, consumers are enabled to see the absorbent articles 1004 enclosed in the package.

In one example, the second package material may have a higher Basis Weight than the first package material according to the Basis Weight Test Method disclosed herein. In particular, the second package material may have a basis weight of from 90 gsm to 120 gsm, in particular from 95 gsm to 115 gsm and the first package material may have a basis weight from 50 gsm to 89 gsm, in particular from 60 gsm to 80 gsm. Due to the higher basis weight, the second sheet of second package material has a higher tendency to return to the closed position after opening of the consumer and thus reclosing and handling of the package is facilitated.

The package materials of the present disclosure comprise natural fibers. The package materials of the present disclosure may comprise wood fiber and/or pulp fiber. The package materials may comprise at least 50 percent by weight natural fibers, at least 70 percent by weight natural fibers, at least 90 percent by weight natural fibers, between about 50 percent and about 100 percent by weight natural fibers, between about 65 percent and about 99 percent by weight natural fibers, or between about 75 percent and about 95 percent by weight of natural fibers, specifically reciting all values within these ranges and any ranges formed therein or thereby. In one form, the package materials may comprise 99.9% percent by weight natural fibers. By having such content of natural fibers, the package material may exhibit sufficient strength to not collapse upon opening and removal of one or more absorbent articles 1004.

Inks and/or dyes associated with the package art, branding, package information, and/or background color, as well as adhesives associated with the seams and barrier coatings are also considered part of the package materials on a weight percentage basis. Where the weight percentage of natural fibers is less than 100 percent, the difference may be made up by inks, dyes, and/or adhesives. Inks, dyes, coatings, and adhesives may be considered contaminants in a paper recycling process, but may be otherwise recyclable.

While the package materials may comprise many different fibers, inks, dyes, coatings, adhesives, etc., the package material of the present disclosure may be constructed to facilitate and/or encourage recycling of the package material, and may encourage recycling of the package material within a single recycling stream, such as a paper recycling stream. Whether package materials are recyclable may vary from region to region. In order to meet one of the highest standards for recyclability, the total weight percentage of non-recyclable material, including material not recyclable within a particular recycling stream - such as a paper recycling stream - but otherwise recyclable, e.g. inks, dyes, adhesives, and coatings in the package material may be 5 percent by weight of the package material or less, or between 0.1 percent to 5 percent by weight, specifically reciting all values within these ranges and any ranges formed therein. However, other jurisdictions may allow a higher weight percentage of non-recyclable material. For example, in other jurisdictions, the package material of the present disclosure may comprise 50 percent by weight or less, 30 percent by weight or less, or about 15 percent by weight or less of non-recyclable material, specifically including all values within these ranges and any ranges formed therein or thereby. As another example, the package materials of the present disclosure may comprise from between about 0.1 percent to about 50 percent by weight, from about 0.1 percent to about 30 percent by weight, or from about 0.1 percent to about 15 percent by weight of non-recyclable material, specifically including all values within these ranges and any ranges formed therein or thereby. In an example, the amount of inks, dyes, coatings, and adhesives is 5 percent by weight, or less, or between 0.1 percent by weight to 5 percent by weight, specifically reciting all values within these ranges and any ranges formed therein.

The package materials of the present disclosure may be free of a barrier layer. As used herein, the term "barrier layer" refers to a layer of material, including barrier coatings, barrier plastics, and/or barrier foils, that is joined to the package materials comprising natural fibers. Such barrier layers may reduce the recyclability of the package materials within a single recycling stream.

In other instances, in order to at least partially protect absorbent articles disposed within the package, the package materials of the present disclosure may comprise a barrier layer. The barrier layer may at least partially inhibit the migration of water vapor through the package material. The barrier layer may comprise a water soluble material that may not interfere with a recycling process. The barrier layer may be easily separable from the remainder of the package materials through a recycling process, for example by having a different water solubility, density, buoyancy, or other physical features as compared to the remainder of the package materials.

Where a barrier layer is utilized, the barrier material may be selected such that the use of adhesives can be reduced or eliminated. One such barrier material may be polyethylene film coated on an inner surface of the package material. The polyethylene may be utilized to form the seals rather than an adhesive or in conjunction with an adhesive. However, as the polyethylene film may not be recyclable in the same stream as the other package materials, the weight percentage of the polyethylene may be in accordance with the present description regarding percentages of non-recyclable material discussed herein.

The basis weight of the one or more barrier film layers should be at least about 2 gsm to less than about 25 gsm. It is theorized that below this basis weight, the film may not cover a sufficient enough portion of the bag to provide a suitable barrier property. However, aside from the foregoing, the one or more barrier film layers may be any suitable basis weight so long as the one or more barrier film layers' basis weight is within the weight percentages described herein.

It is worth noting that the one or more barrier film layers laminated, coated, or otherwise joined to the natural fiber layer forms a synergistic relationship particularly where the one or more barrier film layers are desired to be a smaller weight percentage of the overall first and/ or second and/ or third package material. For example, where the one or more barrier film layers is 5 percent by weight or less of the overall first and/ or second and/ or third package material, this can be a very small basis weight barrier film layer for the basis weight of package materials described heretofore. At this very low basis weight, it is believed that the one or more barrier film layers would not be able to be processed reliably without being coated, laminated, or otherwise joined to the natural material layer. And similarly, without the addition of the one or more barrier film layers, the natural material layer may not be able to provide much, if any, inhibition to the absorption of moisture vapor by the SAP within the absorbent articles in the package.

Regarding a method by which the one or more barrier film layers may be joined to the natural fiber layer, an example method is described hereafter. The one or more barrier film layers can be a polymer that is water insoluble. The polymer may be obtained from a manufacturer as a pre-made dispersion/emulsion of the polymer, or a dispersion/emulsion may be formed if a pre-made dispersion/emulsion is not available. The aqueous polymeric system is then coated onto the natural fiber layer and the water (or other solvent e.g. alcohol) may then be removed via convective or diffusive drying process. Afterwards, enough heat may be applied to form a continuous polymeric layer.

Without being limited to theory, it is believed that the most important material properties of the aqueous polymeric system are: a) the ability of the polymer to be made into an emulsion in water; b) the resulting viscosity of the aqueous polymeric system at that temperature, higher viscosity being better for maximum distinction / separation between the layers; c) the wetting of the aqueous polymeric system either onto a substrate to be coated, higher wetting being better.

Another example involves thermal extrusion coating. Thermal extrusion coating is used to apply a composition that is not water-borne. In this method, the polymeric composition may be melted within an extruder; the molten polymeric composition is thermally extruded onto the surface of the natural fiber layer followed by cooling to form the package material.

In yet another example, the one or more barrier film layers may be applied to the natural fiber layer via adhesive lamination. If such an execution is performed, care should be taken regarding the type of adhesive as well as the amount of adhesive used as this could impact the overall recyclability of the package material. If such an arrangement was used, an adhesive layer can be applied directly to the natural fiber layer, and a pre-made barrier film layer or layers would then be applied to the adhesive layer. A polymeric composition can be made into a pre-made barrier film layer or layers by a variety of methods including solution casing, thermal cast film extrusion and thermal blown film extrusion. In yet another example, heat lamination may be used to adhere the polymeric barrier film layer or layers to the natural fiber layer.

Regarding suitable polymeric compositions that can be utilized in the one or more barrier film layers, there are many that could be either biodegradable or could be non-biodegradable. Some examples of biodegradable options include aliphatic aromatic polyesters (e. g., ECOFLEX^{®} from BASF), certain thermoplastic starches (e.g., MATER-BI from Novamont's or PLANTIC^{®} from Plantic/Kuraray), polybutylene succinate and copolymers thereof (e.g., BIONOLLE^{®} from ShoWa High polymer Co. or PBSA from Mitsubishi Chemicals), polycaptralactone and mixtures thereof. Other suitable polymers include polhydroyxalkoanates (PHA) and PHA copolymers such as poly(beta-hydroxyalkanoate), poly(3-hydroxybutyrate-co-3-hydroxyhexanoate) NODAXTM from Danimer, and poly(3-hydroxybutyrate-co-3-hydroxyhexanoate) from Kaneka. Non-limiting examples of PHA copolymers include those described in US. Pat. No. 5,498,692. Other PHA copolymers can by synthesized by methods known to one skilled in the art, such as, from microorganisms, the ring-opening polymerization of beta-lactones, the dehydration-polycondensation of hydroxyalkanoic acid, and the de-alcoholization-polycondensation of the alkyl ether of hydroxyalkanoic acid, as described in Volova, "Polyhydroxy Alkanoates Plastic Materials of the 21" Century: Production, Properties, and Application, Nova Science Publishers, Inc., (2004), incorporated herein by reference. Another example is polylactic acid (PLA). Additional examples of non-biodegradable options include polyolefin materials, for example: polyethylene (PE), polypropylene (PP), and polyethylene terephthalate (PET). Examples of polyethylene could include high density polyethylene, low density polyethylene, linear low density polyethylene, medium density polyethylene - including all homopolymers and copolymers of those materials. Other examples of non-biodegradable options could include various Surlyn's; copolymers of styrene-butadiene e.g. acrylo-nitrile-butadiene; acrylic copolymers; acrylate copolymers (including methyl methacrylate); acetate copolymers including EVA (ethylene vinyl acetate). The polymers could in some cases include fillers additives such as clays (e.g. kaolin) and other mineral additives such as CaCO3 or TiO2.

Ideally, the package materials of the present disclosure would provide unlimited protection inhibiting the absorption of moisture vapor by the SAP in the absorbent articles therein. However, as explained previously, the addition of one or more barrier film layers to the natural fibers can negatively impact the recyclability of the natural fibers. So, careful selection of the one or more barrier film layers and thus the package material should be taken.

Instead of having a 100% coating of LDPE across the entire inner surface of the first and/ or second and/ or third package material, only certainly regions may have the LDPE. HDPE has more desirable barrier properties than LDPE, but LDPE has more desirable sealing and/or seaming properties. As such, it may be desirable to include the HDPE at a higher basis weight across all of, or most of, the inner surface of the packaging material and provide the LDPE only at seams and/or sealing areas where the LDPE has the most functionality. As an example, the inner surface of the first and/ or second and/ or third package packaging material may be coated with 10 g of HDPE at 100% area of the inner surface and 6 g of LDPE at about 5% to about 35% area, about 5% to about 25% area, about 10% to about 20% area, or about 15% area of the inner surface of the package material. The LDPE may be placed in areas of the seams or where seaming or sealing occurs. Stated different, the LDPE may not overlap all of the HDPE. This would allow the package to have improved barrier properties, with adequate seaming and/or sealing properties, and while still meeting the recyclability guidelines. Instead of LDPE being present in the sealing and/or seaming areas, other bonding enhancement agents with desirable heat sealability and sufficient seal and/or seam strength may be used. An example of a bonding enhancement agent is a heat sealable adhesive. Bonding enhancement agents may be flexo-printed, dispersion coated, applied by ink jet printing, or applied using any other specific application technology.

Instead of using HDPE and LDPE as the barrier and/or sealing materials, the natural fibers may be coated with clay, hectorite, and/or PET that is not sealable, but does provide a suitable barrier. Another option would be to join metalized paper, or other inorganic barrier material, to the natural fibers, which again provides a suitable barrier. Aluminiumoxide barriers are also contemplated to be joined the natural fibers. The Aluminiumoxide barriers are transparent which may be desirable and/or less noticeable to consumers. With all of these options, bonding enhancement agents may be applied to seal and/or seam areas on the inner surface of the package material to allowed seals or seams to be formed when the package is made even though the initial barrier materials are not sealable. In general, the clay, hectorite, PET, metalized paper, inorganic barriers, and/or aluminiumoxide, including the bonding enhancement agents in the seal or seam areas may be less than about 15%, less than about 10%, less than about 5%, about 2% to about 10%, or about 3% to about 8%, by weight of the laminate of these materials and the natural fibers to meet recycling requirements.

The effectiveness of the recycling process on the package material of the present disclosure may be determined via recyclable percentage. Package material of the present disclosure may exhibit recyclable percentages of 60 percent or greater, 75 percent or greater, or 90 percent or greater, specifically reciting all values within these ranges and any ranges formed therein or thereby. The packaging material of the present disclosure may have a recyclable percentage of between about 60 percent and about 99.9 percent, between about 75 percent and about 99.9 percent, or between about 90 percent and about 99.9 percent, specifically reciting all values within these ranges and any ranges formed therein or thereby. In a specific example, the package material of the present disclosure may exhibit a recyclable percentage of between about 95 percent and about 99.9 percent, specifically including all values within these ranges and any ranges formed therein. The recyclable percentage of the package material of the present disclosure is determined via test PTS-RH:021/97 (Draft Oct. 2019) under category II, as performed by Papiertechnische Stiftung located at Pirnaer Strasse 37, 01809 Heidenau, Germany.

Along with recyclable percentage, the total reject percentage is determined via the PTS-RH:021/97 (Draft Oct. 2019) under category II Test Method. The total reject percentage of the package material of the present disclosure may be 40 percent or less, 30 percent or less, or 10 percent or less, specifically including all values within these ranges and any ranges formed therein or thereby. For example, the total rejection percentage of the package material of the present disclosure may be from about 0.5 percent to about 40 percent, from about 0.5 percent to about 30 percent, or from about 0.5 percent to about 10 percent, specifically reciting all values within these ranges and any ranges formed therein or thereby.

It is believed that the percent non-recyclable material does not necessarily have a 1:1 correlation to the total reject percentage. For example, dissolvable adhesives and/or coatings are designed to dissolve during the recycling process. It is theorized that these adhesives may not have an impact the total reject percentage; however, they would contribute to the non-recyclable material weight percent.

The PTS-RH:021/97 (Draft Oct. 2019) under category II Test Method also comprises a visual component. Trained screeners inspect one or more handsheets of recycled package material for visual imperfections. If the number of visual imperfections is too great, then the package material is rejected. If the number of visual imperfections is acceptable, in accordance with the PTS-RH:021/97 (Draft Oct. 2019) under category II Test Method, then the package material is approved for additional processing. The package material of the present disclosure may yield an acceptable level of visual imperfections during this step of the method.

The package material of the present disclosure may yield the recyclable percentages mentioned heretofore as well as pass the visual screening method. Thus, the package material of the present disclosure may achieve an overall score or final outcome of "pass" when subjected to the PTS-RH:021/97 (Draft Oct. 2019) under category II Test Method.

It is also worth noting that there is an alternative method for determining the recyclable percentage of the package material of the present disclosure. The Test Method performed by the University of Western Michigan, called the Repulpability Test Method, may provide a percent yield of recyclable material. While there are subtle differences between the Repulpability Test Method performed by Western Michigan and the PTS-RH:021/97 (Draft Oct. 2019) under category II Test Method, it is believed that the percentage yield of the Repulpability Test Method would be similar to the recyclable percentage provided by the PTS Test Method.

It is contemplated that the package materials of the present disclosure, while being recyclable, may itself comprise recycled material. Such determination can be made from a visual inspection of the package. For example, manufacturers typically advertise the use of recycled materials in an effort to demonstrate their eco-friendly product approach. To further expand on this example, some manufacturers may utilize a logo, e.g., a leaf, along with wording to indicate the use of recycled material in the package material. Often times, manufacturers may specify the percentage of recycled material utilized as well, e.g., over 50 percent, over 70 percent, etc.

Visual inspection may be as simple as utilizing the human eye to inspect packages for logos of the use of recycled material. Additionally, or alternatively, visual inspection may include microscopy methods such as optical microscopy, scanning electron microscopy or other suitable methods known in the art. For example, package material comprising recycled paper fibers may appear different under a microscope due to the presence of a much wider range of natural fiber types than if the package material comprised of 100% non-recycled paper. As another example, under a microscope, recycled fibers - due to their increased processing - may appear more fibrillated than their virgin fiber counterparts.

In order to withstand the rigors of a high speed manufacturing process where a plurality of absorbent articles are placed within the package, withstand the force of compressed absorbent articles being placed directly into the package without an intermediate package or container, withstand the rigors of being shipped, provide protection from environmental insults during shipping and while on the store shelf, and provide for product protection while in the consumers home, the package materials may have some level of strength, stretch, and/or resilience. The package materials of the present disclosure may be characterized using metrics such as: MD Tensile Strength in kN/m, CD Tensile Strength in kN/m, MD Stretch At Break in percent, CD Stretch At Break in percent, Burst Strength in kPa, Caliper in µm, MD Tensile Energy Absorption in J/m², CD Tensile Energy Absorption in J/m², and Basis Weight in grams per square meter. While all of the metrics may be utilized together to characterize the package materials of the present disclosure, it is believed that some of the metrics alone or in conjunction with others may suffice to characterize package materials which are suitable for packaging absorbent articles. As an example, it is believed that the Burst Strength may be utilized alone or in conjunction with other metrics to obtain package materials which are sufficient for packaging of absorbent articles. Similarly, it is believed that the Tensile Energy Absorption (TEA) in the MD and CD may be utilized in conjunction with one another, and if desired, along with any other combination of the above metrics, to obtain package materials which are suitable for packaging of absorbent articles. As yet another example, it is contemplated that MD Stretch At Break and/or CD Stretch At Break may be utilized in conjunction with at least one of MD Tensile Strength or CD Tensile Strength, respectively, to characterize package materials which may be sufficient to package absorbent articles as described herein. Any suitable combination of metrics may be utilized.

The package materials of the present disclosure may have an MD Tensile Strength of at least 5 kN/m, at least 7 kN/m, or at least 8 kN/m, specifically reciting all values within these ranges and any ranges formed therein or thereby. The MD Tensile Strength may be between about 5 kN/m and about 8.5 kN/m, between about 5.2 kN/m and about 8.2 kN/m, or between about 5.5 kN/m and about 8.0 kN/m, specifically reciting all values within these ranges and any ranges formed therein or thereby. The MD Tensile Strength is measured using the Strength Tensile Test Method described herein.

The package materials of the present disclosure may have a CD Tensile Strength of at least 3 kN/m, at least 4 kN/m, or at least 5.5 kN/m, specifically reciting all values within these ranges and any ranges formed therein or thereby. The CD Tensile Strength may be between about 3 kN/m and about 6.5 kN/m, between about 3 kN/m and about 6.2 kN/m, or between about 3 kN/m and about 6 kN/m, specifically reciting all values within these ranges and any ranges formed therein or thereby. The CD tensile strength is measured using the Strength Tensile Test Method.

The package materials of the present disclosure may have a Burst Strength of at least 200 kPa, at least 250 kPa, or at least 550 kPa, specifically reciting all values within these ranges and any ranges formed therein or thereby. The Burst Strength of the package materials of the present disclosure may be between about 200 kPa and about 600 kPa, between about 220 kPa and about 550 kPa, or between about 250kPa and about 500 kPa, specifically reciting all values within these ranges and any ranges formed therein or thereby. The Burst Strength is measured using the Burst Strength Test Method described herein. It is believed that the Burst Strength, as measured, includes components of strength, flexibility, and resiliency. As such, it is believed that Burst Strength may be used independently from the other metrics mentioned.

The package materials of the present disclosure, in addition to strength, may also exhibit some measure of resiliency. Thus, the package materials of the present disclosure may exhibit an MD Stretch At Break of at least 3 percent, at least 4 percent, or at least 6 percent, specifically reciting all values within these ranges and any ranges formed therein or thereby. The package materials of the present disclosure may exhibit an MD Stretch At Break of between about 3 percent and about 6.5 percent, between about 3.2 percent and about 6.2 percent, or between about 3.5 percent and about 6 percent, specifically reciting all values within these ranges and any ranges formed therein or thereby. The MD Stretch At Break is measured using the Strength Tensile Test Method described herein.

The package materials of the present disclosure may exhibit a CD Stretch At Break of at least 4 percent, at least 6 percent, or at least 9 percent, specifically reciting all values within these ranges and any ranges formed therein or thereby. The package materials of the present disclosure may exhibit a CD Stretch At Break of from about 4 percent and about 10 percent, from about 4.5 percent and about 9.5 percent, or from about 5 percent and about 9 percent, specifically reciting all values within these ranges and any ranges formed therein or thereby. The CD Stretch At Break is measured using the Strength Tensile Test Method described herein.

Regarding Caliper, the package materials of the present disclosure may exhibit a Caliper of at least 50 µm, at least 70 µm, or at least 90 µm, specifically reciting all values within these ranges and any ranges formed therein or thereby. The package materials of the present disclosure may exhibit a Caliper of between about 50 µm and about 110 µm, from about 55 µm and about 105 µm, or from about 60 µm and about 100 µm, specifically reciting all values within these ranges and any ranges formed therein or thereby. Caliper is measured using the Caliper Test Method described herein.

Regarding Tensile Energy Absorption (TEA), the package materials of the present disclosure may exhibit an MD TEA of at least 150 J/m², greater than 170 J/m², or at least 180 J/m², specifically reciting all values within these ranges and any ranges formed therein or thereby. The package materials of the present disclosure may have an MD TEA of between about 100 J/m² and about 250 J/m², between about 125 J/m² and about 225 J/m², or between about 150 J/m² and about 200 J/m², specifically reciting all values within these ranges and any ranges formed therein or thereby.

The package materials of the present disclosure may have a CD TEA of at least 150 J/m², at least 200 J/m², or at least 250 J/m², specifically reciting all values within these ranges and any ranges formed therein or thereby. The package materials of the present disclosure may have a CD TEA of between about 150 J/m² and about 275 J/m², from about 175 J/m² and about 260 J/m², or between about 200 J/m² and about 250 J/m², specifically reciting all values within these ranges and any ranges formed therein or thereby. TEA in the MD and CD are measured according the Strength Tensile Test Method described herein.

The Basis Weight of the package materials may affect the "feel" of the package to the consumer as well as the strength of the package. Too low of a Basis Weight and the package may feel too flimsy. Too high and the package may feel too inflexible. The package materials of the present disclosure may have a Basis Weight of between about 50 gsm and about 120 gsm, between about 55 and about 115 gsm, or between about 60 gsm and about 110 gsm, specifically reciting all values within these ranges and any ranges formed therein or thereby. By such Basis Weights it is ensured that the package is flexible but retains its shape after opening and removal of one or more absorbent articles 1004. The Basis Weight, also referred to as "grammage", is determined according to the Basis Weight Test Method described herein.

It is worth noting that for high speed packaging processes, the lower Basis Weight of 50 gsm may provide some quality assurance outages. It is believed that high speed packaging processes may cause strain on the packaging materials that slower packaging processes may not. Therefore, where package materials are processed using a high speed manufacturing process, 60 gsm may be the lowest desirable package material Basis Weight. Where package materials are processed using a hand packing process or lower speed packaging processes, 50 gsm may be sufficient as the lowest package material Basis Weight.

The package materials of the present disclosure are different than carton board and cardboard. For example, carton board is not as flexible as the package materials of the present disclosure. Carton board is inherently stiffer than the package materials of the present disclosure and does not have the processability on high speed converting lines as does the package materials of the present disclosure. Additionally, carton board has a Basis Weight greater than 160 gsm, which is considerably higher than that of the package materials of the present disclosure.

Similarly, cardboard is also different than the package materials of the present disclosure. Cardboard has a much higher Basis Weight (greater than 200 gsm) than those of the package materials of the present disclosure. Additionally, cardboard is much less flexible than the package materials of the present disclosure. Cardboard materials are commonly fluted and comprise three plies of a paper material and, as such, are structurally different than the package materials of the present disclosure.

The package materials of the present disclosure have the advantage of being more flexible as compared to carton board and cardboard. Another advantage is that the package materials of the present disclosure take up less space than the more-bulky carton board and cardboard. A further advantage of the package materials of the present disclosure, attributable at least in part to the strength and resiliency properties discussed herein, is that the package materials allow the packaged absorbent articles to be compressed within the package. This allows for more products to fit within a smaller volume package which may increase manufacturing efficiency. One additional advantage is that a single layer (one ply) of the package materials of the present disclosure may form packages of the present disclosure. The inventors have found that, due at least in part to the flexibility, strength, and resiliency properties of the package materials, packages of the present disclosure may be formed from a single layer (one ply) of package materials of the present disclosure.

Despite having reduced flexibility compared to, for example, plastic packaging, and lower Basis Weight than cardboard and carton board, the inventors have surprisingly found the packaging materials of the present disclosure may withstand the rigors of a high speed manufacturing process - where a plurality of absorbent articles are placed within the package under compression - as well as the rigors of being shipped, provide protection from environmental insults during shipping and while on the store shelf, and provide protection for absorbent articles while in the consumers home.

Table 1 shows a variety of package materials which are able to be successfully utilized in packaging absorbent articles under high speed processing conditions, along with at least one package material which is not successful. The various properties discussed previously are also listed for each of the samples.

Sample 1: Packaging paper produced from pure, white kraft pulp and consisting entirely of virgin fibers, available from BillerudKorsnäs^{™} under the trade name Axello Tough White.

Sample 2: Packaging paper produced from pure, white kraft pulp and consisting entirely of virgin fibers, available from BillerudKorsnäs^{™} under the trade name Performance White SE.

Sample 3: Calendered specialty kraft paper consisting entirely of virgin fibers, available from Mondi^{™} under the trade name Advantage Smooth White Strong.

Sample 4: Packaging paper produced from kraft pulp, made of virgin fibers, and comprising a barrier coating of fluoropolymers, available from BillerudKorsnäs^{™} under the trade name Basix Glaze.

**Table 1**

| | Sample 1 | Sample 2 | Sample 3 | Sample 4 |
|---|---|---|---|---|
| Basis Weight (gsm) | 80 | 70 | 70 | 50 |
| MD Tensile Strength(kN/m) | 7.6 | 5.7 | 5.9 | 4.7 |
| CD Tensile Strength (kN/m) | 4.7 | 4.1 | 3.0 | 2.7 |
| Burst Strength (kPa) | 480 | -- | 256 | 185 |
| MD Stretch At Break (%) | 4.5 | 6.0 | 2.5 | -- |
| CD Stretch At Break (%) | 8.0 | 9.5 | 8.0 | -- |
| Caliper (µm) | 92.0 | -- | 89.0 | 67.0 |
| TEA MD (J/m²) | 185 | 230 | -- | -- |
| TEA CD (J/m²) | 240 | 200 | -- | -- |

The package material of Sample 4 is not able to be successfully utilized in the packaging of absorbent articles. During the placement of absorbent article in the package, the package material tore. Without wishing to be bound by theory, it is believed that Sample 4 failed due to a combination of low Basis Weight and a high speed packaging process. While Sample 4 failed under the conditions of the high speed process, it is believed that a sample having the properties of Sample 4 may be successful with the use of a gentler packaging process, such as hand packing.

### Absorbent Article Configuration

The packages of the present disclosure may comprise one or more absorbent articles. The absorbent articles may be placed into the package in an unfolded or folded configuration. The articles may be folded laterally and/or longitudinally. The articles may comprise one fold line, and may be disposed within the package in a bi-fold configuration. The articles may comprise two fold lines, and may be disposed within the package in a tri-fold configuration.

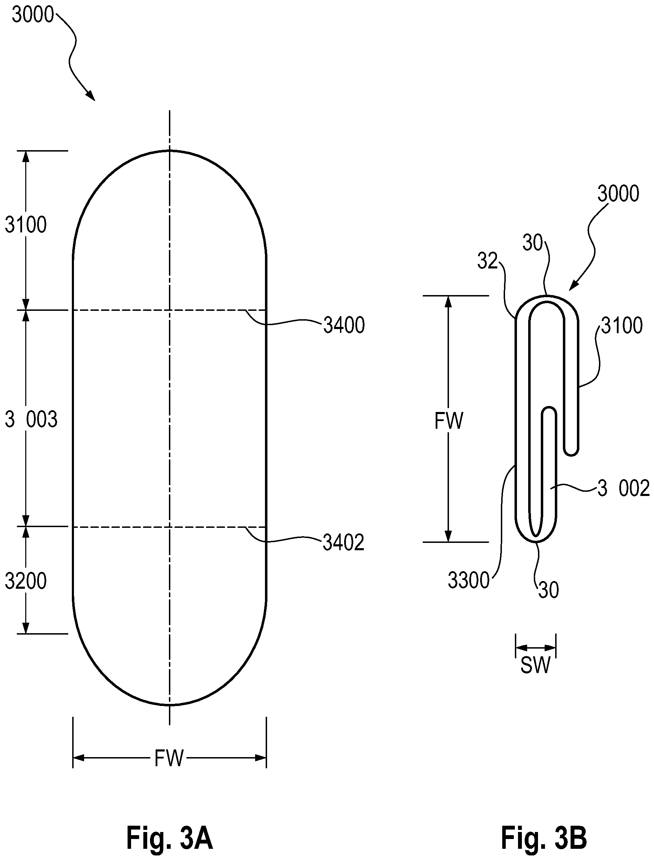

Fig. 3A depicts an example of a feminine hygiene pad in an unfolded configuration. Fig. 3B depicts a side view of the feminine hygiene pad of Fig. 3A in a tri-fold configuration. The feminine hygiene pad depicted in Figs. 3A and 3B comprises a first fold line 3400 disposed between a first end region of the pad 3100 and a central region of the pad 3300, and a second fold line 3402 disposed between a second end region of the pad 3200 and the central region 3300. Prior to placement within the package, the second end region 3200 may be folded over and longitudinally inward about the second fold line 3402 to overlap at least a portion of the central region 3300, as may be appreciated from a comparison of Figs. 3A and 3B. The first end region 3100 may then be folded over and longitudinally inward about the first fold line 3400 to overlap at least a portion of the central region 3300 and a portion of the second end region 3200. In some examples a tri-fold configuration may have the article folded approximately in thirds, about the two longitudinally-spaced lateral fold lines.

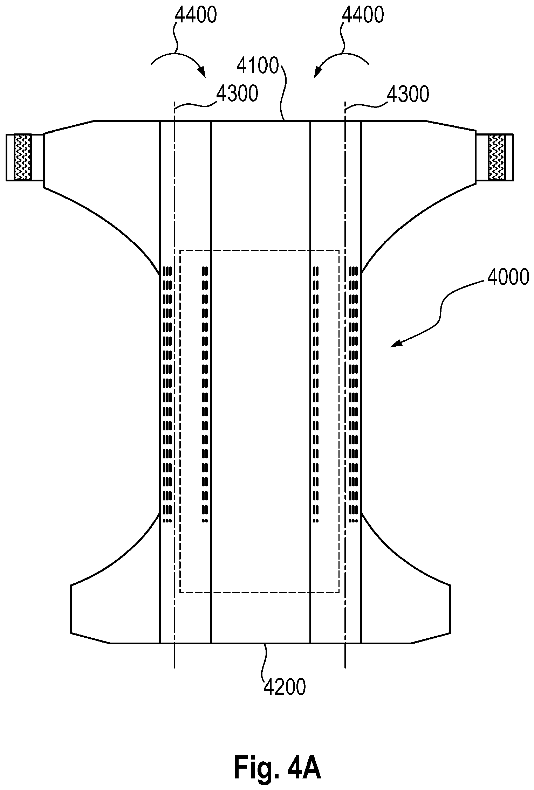

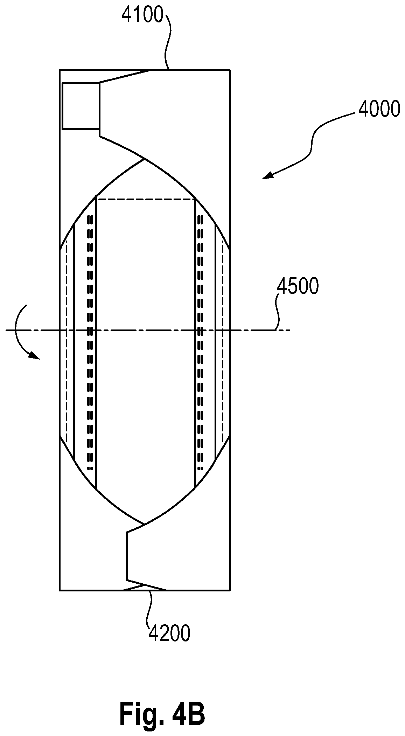

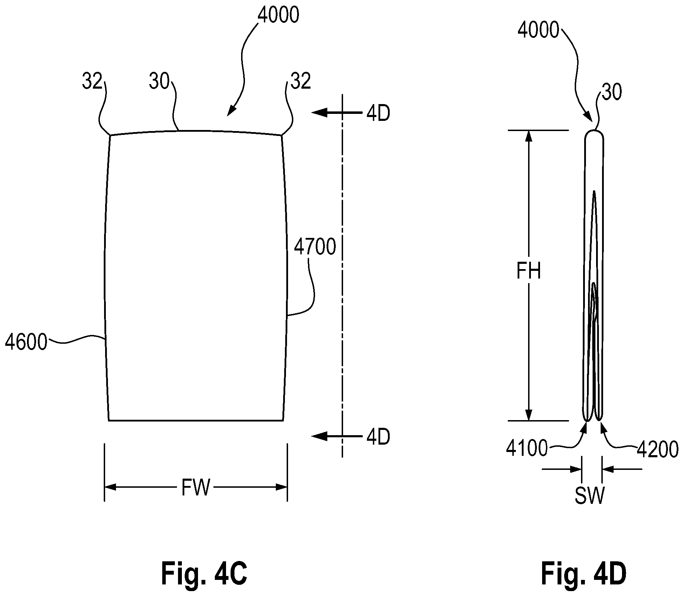

Figs. 4A-4D depict an absorbent article in the form of a diaper 4000 with front and rear waist edges 4100, 4200, in successively open/unfolded and folded configurations. For packaging in bulk, each of a plurality of diapers such as that shown in Fig. 4A may, in a possible first step, have its longitudinal side portions be folded over and laterally inward about longitudinal side edge fold lines 4300, as may be appreciated from a comparison of Figs. 4A and 4B. Next, the diaper may, in a second step, be folded longitudinally, about lateral fold line 4400 that passes through the crotch region of the diaper, as may be appreciated from a comparison of Figs. 4B and 4C. For a bi-fold configuration such as depicted in Figs. 4C and 4D, the article may be folded longitudinally once, and may in some examples be folded approximately in half about the lateral fold line 4400.

Regardless of whether the article is in a bi-fold or tri-fold configuration, the folded article, such as folded feminine hygiene pad 3000 and/or folded diaper 4000, may have a single fold nose 30 defining at least one end edge of the folded article, fold nose corners 32, and left and right longitudinal peripheral edges 4500, 4600. It will be appreciated that in a tri-fold example, a single fold nose may define each of both end edges of the folded article. In some examples, such as depicted in Figs. 4C and 4D, fold nose 30 may be proximate the crotch region of the article (the middle region of the article adapted to be located between the wearer's legs during wear). The folded article will have a folded width FW measured as the distance between side edges, a folded height FH measured as the distance between fold nose 30 and the end edges (in the case of a bi-fold configuration) or between the two fold noses 30 (in the case of a tri-fold configuration), and a side width (SW). The folded width (FW) forms the flat, broad face of the folded absorbent article, while the side width (SW) forms the narrow folded side of the absorbent article.

A plurality of folded articles such as depicted in Figs. 4B and 4C and 4D may be placed in similar orientation within the interior compartment of a package of the present disclosure. One or more absorbent articles are disposed within a package of the present disclosure. The package defines an interior compartment in which a plurality of absorbent articles 1004 are situated. The plurality of absorbent articles 1004 may be arranged in a single horizontal row or in one or more vertical stacks. Where the articles are arranged in more than one vertical stack all the articles may be oriented in the same direction. In another example, a first set of the plurality of folded articles may have their fold noses 30 oriented along one side of the stack, and a second set of the plurality of folded articles may be rotated 180 degrees to have their fold noses oriented along the opposite side of the stack. In some examples, the articles in the first set and the articles in the second set may appear in alternating sequence in the stack.

The folded absorbent articles may be disposed within the package of the present disclosure such that the folded width (FW) faces toward the first and second side panels. Such a configuration may be employed where the number of absorbent articles within the package is relatively large, e.g., greater than about ten individual absorbent articles, because the narrower sides (SW) of the articles will form front and back panels. Therefore, a relatively large number of absorbent articles may then be utilized to build up the front and back panels of the package. Such a configuration may be beneficial where the front and/or back panels form the consumer-facing panel.

The folded absorbent articles may be disposed within the package of the present disclosure such that the folded width (FW) faces toward the front and back panels. Such a configuration may be employed where the number of absorbent articles within the package is relatively low, e.g., less than about ten individual absorbent articles, because the wider sides (FW) of the articles will form front and back panels. Such a configuration may be beneficial where the front and/or back panels form the consumer-facing panel, and the number of absorbent articles disposed within the package is less than about ten.

The absorbent articles or articles may be packed under compression so as to reduce the size of the package, while still providing an adequate number of absorbent articles per package. By packaging the absorbent articles under compression, caregivers can easily handle and store the packages, while also providing distribution savings to manufacturers owing to the reduced size of the packages. Despite lacking the stretch properties of conventional plastic packaging material, the inventors have surprisingly found the package materials of the present disclosure are able to withstand the processing and distribution rigors, as mentioned herein, even with absorbent articles which are compressed within the package and without the use of an intermediate container. This is particularly unexpected as the materials of the present disclosure may not display the stretch properties of presently used conventional plastic films.

Packages of absorbent articles of the present disclosure may have an In-Bag Stack Height of less than about 150 mm, less than about 110 mm, less than about 105 mm, less than about 100 mm, less than about 95 mm, less than about 90 mm, less than about 85 mm, less than about 80 mm, less than about 78 mm, less than about 76 mm, less than about 74 mm, less than about 72 mm, or less than about 70 mm, specifically reciting all 0.1 mm increments within the specified ranges and all ranges formed therein or thereby, according to the In-Bag Stack Height Test described herein. Alternatively, packages of the absorbent articles of the present disclosure may have an In-Bag Stack Height of from about 70 mm to about 150 mm, from about 70 mm to about 110 mm, from about 70 mm to about 105 mm, from about 70 mm to about 100 mm, from about 70 mm to about 95 mm, from about 70 mm to about 90 mm, from about 70 mm to about 85 mm, from about 72 mm to about 80 mm, or from about 74 mm to about 78 mm, specifically reciting all 0.1 mm increments within the specified ranges and all ranges formed therein or thereby, according to the In-Back Stack Height Test Method described herein.

### Other Products within the Packages of the Present Disclosure

Many suitable products may be placed within the packages and package materials of the present disclosure, such as consumer products. As an example, packages of the present disclosure may comprise or have contained therein, one or more moisture labile products, humidity labile products, moisture sensitive products, or water vapor sensitive products including unit dose products, unit dose pouches, articles meant for single use, pouches, pouches with fibrous wall materials, pouches with soluble film wall materials, pouches comprising a single layer or ply, and combinations thereof. These unit dose or pouch form products are delivery vehicles comprising active agents or additives designed and intended to provide a benefit to something, such as providing a benefit to an environment external to the unit dose or pouch. One embodiment may comprise a unit dose article or pouch where active agents are contained on the internal volume of the unit dose article or pouch. Another embodiment may comprise a unit dose or pouch comprising a single layer or ply where the active agent is contained within the single layer or ply, coated or embedded on the surface of the layer or ply, or a combination of these two configurations.

Active agents may be any suitable additive that produces an intended effect under intended use conditions of the unit dose article or pouch. For example, the active agent may be selected from the group consisting of: personal cleansing and/or conditioning agents, such as hair care agents such as shampoo agents and/or hair colorant agents, hair conditioning agents, skin care agents, sunscreen agents, and skin conditioning agents; laundry care and/or conditioning agents such as fabric care agents, fabric conditioning agents, fabric softening agents, fabric anti-wrinkling agents, fabric care anti-static agents, fabric care stain removal agents, soil release agents, dispersing agents, suds suppressing agents, suds boosting agents, anti-foam agents, and fabric refreshing agents; liquid and/or powder dishwashing agents (for hand dishwashing and/or automatic dishwashing machine applications), hard surface care agents, and/or conditioning agents and/or polishing agents; other cleaning and/or conditioning agents such as antimicrobial agents, antibacterial agents, antifungal agents, fabric hueing agents, perfume, bleaching agents (such as oxygen bleaching agents, hydrogen peroxide, percarbonate bleaching agents, perborate bleaching agents, chlorine bleaching agents), bleach activating agents, chelating agents, builders, lotions, brightening agents, air care agents, carpet care agents, dye transfer-inhibiting agents, clay soil removing agents, anti-redeposition agents, polymeric soil release agents, polymeric dispersing agents, alkoxylated polyamine polymers, alkoxylated polycarboxylate polymers, amphilic graft copolymers, dissolution aids, buffering systems, water-softening agents, water-hardening agents, pH adjusting agents, enzymes, flocculating agents, effervescent agents, preservatives, cosmetic agents, make-up removal agents, lathering agents, deposition aid agents, coacervate-forming agents, clays, thickening agents, latexes, silicas, drying agents, odor control agents, antiperspirant agents, cooling agents, warming agents, absorbent gel agents, anti-inflammatory agents, dyes, pigments, acids, and bases; liquid treatment active agents; agricultural active agents; industrial active agents; ingestible active agents such as medicinal agents, teeth whitening agents, tooth care agents, mouthwash agents, periodontal gum care agents, edible agents, dietary agents, vitamins, minerals; water-treatment agents such as water clarifying and/or water disinfecting agents, surfactants, anionic surfactants, cationic surfactants, nonionic surfactants, zwitterionic surfactants, amphoteric surfactants, one or more silicones, one or more alkali metal or alkaline-earth metal carbonates, alkali metal carbonates (e.g. sodium carbonate, potassium carbonate, etc.), alkali metal hydrogen carbonates (e.g., sodium hydrogen carbonate, potassium hydrogen carbonate, etc.), ammonium carbonate, organic acids (e.g., hydroxy-carboxylic acids [citric acid, tartaric acid, malic acid, lactic acid, gluconic acid, etc.], saturated aliphatic carboxylic acids [acetic acid, succinic acid, etc.], unsaturated aliphatic carboxylic acids [e.g., fumaric acid, etc.]. and mixtures thereof.

Example unit dose articles or pouches of the present disclosure include those described in U.S. Pat. Appl. Pub. No. 2013/0172226, U.S. Pat. Appl. Pub. No. 2015/0071572, U.S. Pat. Appl. Pub. No. 2018/0216285, U.S. Pat. Appl. Pub. No. 2018/0216286, U.S. Pat. Appl. Pub. No. 2018/0216287, U.S. Pat. Appl. Pub. No. 2018/0216288, and PCT publications WO 2022/117853, WO 2022/117854, WO 2022/117855, WO 2022/117856, WO 2022/117858, WO 2022/117859, WO 2022/117860, WO 2022/117861.

### Test Methods

### Seal Strength Test Method

### Reclosable and opening seal strength

The seal strength of the reclosable seal portion and opening area of the reclosable panel on an absorbent article package is determined by measuring the force required to separate one side of the seal (e.g., the second sheet) from the opposing side of the seal (e.g. the first sheet) by executing a typical peel test using a universal constant rate of extension test frame. Seal strength is measured on the reclosable seal portion and opening area of the reclosable panel as it is initially opened (i.e., "first peel"). Additionally, seal strength is measured on the reclosable seal portion of the reclosable panel after it is re-sealed and re-opened for a total of five opening incidents to measure the strength of the reclosable seals. All measurements are performed in a laboratory maintained at 23 °C ± 2 C° and 50% ± 2% relative humidity and test specimens are conditioned in this environment for at least 2 hours prior to testing.

A suitable universal constant rate of extension (CRE) test frame is the MTS Alliance interfaced to a computer running TestSuite control software (available from MTS Systems Corp, Eden Prairie, MN), or equivalent. The CRE test frame is equipped with a load cell for which forces measured are within 1% to 99% of the limit of the cell. The fixtures used to grip the test specimen are clamps with knife or serrated edge grip faces. The grip on the moveable crosshead has a width equal to or greater than the width of the second sheet material of the prepared reclosable panel test sample. The grip on the stationary crosshead has a width equal to or greater than the width of the rigid backing plate, as further described herein. The fixtures are installed on the CRE test frame and mounted such that they are horizontally and vertically aligned with one another.

To support the reclosable panel test sample during the peel test, a rigid backing plate (stainless steel with a thickness of about 1.5 mm) is used. The dimensions of the backing plate are governed by the size of the reclosable panel being tested, as follows. The length of the plate is about 25 mm longer than the overall longitudinal length of the prepared reclosable panel test sample, and the width of the plate is about 10 mm wider than the lateral width of the prepared reclosable panel test sample.

A strip of non-extensible cotton material is secured to the second sheet material at the leading edge (i.e., first opening end; opposite end of the hinge if present) of the reclosable panel test sample to serve as an extension that can reach the upper grip of the tensile tester and enable the reclosable seal to be peeled at 180°. The standard cotton used for this peel test is 100% bleached cotton weave, about 100 g/m² (Style #429W) available from Testfabrics, Inc., West Pittston, PA. Additional distributors of this fabric can be found on the Testfabrics website, www.testfabrics.com. For this execution, the sidedness of the cotton is not relevant. A strip of the standard cotton is cut to a width that is equal to the width of the second sheet material on the prepared reclosable panel test sample with a length that is about 30 mm longer than the longitudinal length of the prepared reclosable panel test sample. The cotton strip must be sufficiently long enough such that after it is secured to the first opening end of the reclosable panel, it can then overlap the entire length of the reclosable panel test sample and still have enough excess leftover to insert into the grip of the tensile tester. A fresh cotton strip is used for each test sample.

A padded weight is used to ensure adequate and reproducible contact between the reclosable seal portion of the first and second sheets of the reclosable panel prior to the initial peal and then for each re-sealing step required for the subsequent peeling incidents. The padded weight consists of a 1/8 inch thick polyurethane foam base (open cell, medium density of 20lbs/cu ft, 18 psi to compress to 25%, available from McMaster-Carr as part #86375K133, or equivalent) plus additional mass required to impart a pressure of about 1.5 psi. The base of the padded weight that will contact the test sample will have dimensions that closely match the dimensions of the reclosable seal portion within the reclosable panel. The foam can be cut to match irregularly shaped seals (i.e., horseshoe-shaped, oval, etc.). Sufficient additional mass is then added on top of the prepared base to reach a pressure of 1.5 psi. The additional mass can be in any convenient form, such as a steel cylinder with washers or shot used to reach the exact mass requirement. For example, a reclosable panel that has a reclosable seal with a width of 25 mm and a length of 20 mm, the 1/8 inch thick polyurethane foam will be cut to form a base that is 25 mm by 20 mm, and the total mass of the prepared base plus additional mass will equal 527.3 g to impart a pressure of 1.5 psi.

A test sample of the reclosable panel of an absorbent article package is prepared and attached to the backing plate as follows. First, the entire reclosable panel that includes the reclosable seals, the hinge, and the permanent seal is excised from the package in a manner that allows for an excess of about 5 mm of the first sheet to extend around the entire perimeter of the second sheet of the reclosable panel. Careful handling of the reclosable panel and test sample is necessary to prevent any disturbance or pre-peeling of any of the seals present. The backing plate is laid flat on a horizontally flat rigid surface. With the second sheet facing up, the excised reclosable panel is laterally centered over the backing plate and the rear longitudinal end of the excised reclosable panel (the end where the hinge is present; opposite the first opening end) is aligned within 1 cm from one of the longitudinal ends of the backing plate (i.e., the end of the plate that will be closest to the grip on the moveable crosshead of the tensile tester). Now the excess edges of the first sheet material that extend along the entire perimeter of the second sheet material of the reclosable panel are secured to the backing plate using very strong one-sided tape (about 1 inch wide; duct tape, or equivalent) as follows. A strip of tape equal in length to the lateral edge of the first sheet material of the reclosable panel is placed over one lateral edge of the first sheet material of the reclosable panel such that it overlaps only the 5 mm of excess first sheet material, with the remaining width of the tape secured to the plate. No portion of the tape is to contact the second sheet material. In like fashion, an additional strip of tape is used to secure the other lateral edge of the first sheet material of the reclosable panel to the plate, with additional strips of tape used to secure both longitudinal edges of the first sheet material of the reclosable panel to the plate. When attached properly, the reclosable panel test sample is held taut without stretching to keep it flat against the backing plate during the peel test, and no wrinkles are present. Now the strip of standard cotton material is attached to the first opening end (i.e., the end opposite the hinge) of the second sheet material of the reclosable panel as follows. A longitudinal edge of the cotton strip is aligned with the longitudinal edge of the first opening end of the second sheet material of the reclosable panel test sample. The cotton strip is secured to the second sheet material using a strip of very strong one-sided tape (about 1 inch wide; duct tape, or equivalent) that has a length equal to the width of the second sheet material and cotton strip. Ensure a good seal between the two materials. To note, for reclosable panel test samples that contain very strong seals, it is necessary to pre-peel a portion of the first opening end of the second sheet material such that a second strip of tape can be applied to the opposing side of the second sheet material to secure it to the cotton strip. The prepared padded weight is then applied to the reclosable seal portion of the reclosable panel test sample that has been attached to the plate for 30 seconds, then removed and set aside. In like fashion, a total of five replicate test samples of the reclosable panel of five separate absorbent article packages are prepared.

Program the CRE test frame for a constant rate of extension uniaxial elongation with a set path length as follows. The gauge length is governed by the length of the backing plate, and it is set to a distance that is about 10 mm greater than the length of the portion of the backing plate that will not be clamped within the grip on the stationary crosshead. The path length is governed by the length of the reclosable seal portion of the test sample. Generally, the path length is set to a distance that is about 5 mm greater than the overall reclosable seal length, however if a hinge or permanent seal is present, the path length can be adjusted such that the extension endpoint occurs prior to any peeling of the permanent seal. The grips are programmed to move closer together (from the zeroed position) by an intentional slack of 1.0 mm to ensure no pretension force exists on the test sample at the onset of the test. The grips are then programmed to move apart at a slack speed of 1.0 mm/s for the initial slack distance of 1.0 mm, then continue to move apart at a speed of 5.0 mm/s until the opposing sides of the reclosable seal portion (i.e., first and second sheets of the reclosable panel) are fully separated and/or the extension endpoint is reached. The grips are then returned to the zero position.

The initial ("first") peel test is executed as follows. Without disturbing the attached strip of cotton, about 10 mm of the longitudinal edge of the backing plate where the first opening end of the reclosable panel test sample is secured is clamped into the grip on the stationary crosshead of the CRE test frame ensuring that no part of the test sample or cotton strip lies within the grip. The position of the backing plate is adjusted, as needed, such that it is centered and parallel to the central pull axis of the CRE test frame. Adjust the distance between the upper and lower grips of the CRE test frame, as needed, to accommodate the backing plate. Now the trailing end of the cotton strip is clamped into the grip of the movable crosshead of the CRE test frame. The position of the cotton strip is adjusted, as needed, such that it is centered and parallel to the central pull axis of the tensile tester. The distance between the grips and the length of the cotton strip clamped within the grip are adjusted, as needed, to minimize the slack of cotton where it is attached to the first opening end of the reclosable panel test sample while ensuring there is ≤ 0.1 N of tension exerted to prevent pre-mature peeling. The crosshead is then zeroed. The peel test is started and force ("load") and displacement data is continuously collected throughout the test at a data acquisition rate of 100 Hz. When the crosshead has returned to the zero position, the backing plate and the cotton strip are removed from the grips in a manner that prevents contamination or distortion to the reclosable seal portion of the reclosable panel test sample. The backing plate is laid flat onto a horizontally flat rigid surface with the reclosable panel test sample facing up. Now the portion of the reclosable seal that was peeled away during the first peel is carefully realigned to match its original position within the reclosable panel. The reclosable seal is then re-sealed by applying the padded weight to the reclosable seal portion of the test sample for 30 seconds. Immediately after 30 seconds have elapsed, the same procedure as previously described for the "first peel" is followed to insert the backing plate and cotton strip into the grips of the CRE test frame, and the second peel test is executed. In like fashion, a third, fourth and fifth peel test is executed for the reclosable panel test sample, re-sealing after each peel test as previously described. After the fifth peel test is complete, the backing plate and cotton strip are removed from the grips and the test sample is discarded. If any of the peeling incidents for a given test sample results in an abnormal peel event (i.e., there is tearing of either sheet material, the adhesive tears or stretches instead of peeling, etc.), all subsequent peeling incidents are aborted for that test sample.

Construct a graph of force (N) versus displacement (mm) for the first peel. From the graph, determine the peak force and record as peak peel force at first peel to the nearest 0.1 N. In like fashion, determine the peak peel force for the second, third, fourth and fifth peels, recording each to the nearest 0.1 N along with the respective second peel, third peel, fourth peel, and fifth peel distinctions.

In like fashion, repeat the entire process until five peels have been executed on all five reclosable panel test sample replicates.

The arithmetic mean of the peak force among the five reclosable panel test sample replicates is calculated for the first peel and reported as Peak Peel Force at first peel to the nearest 0.1 N. In like fashion, the arithmetic mean of the peak force is calculated for the second, third, fourth, and fifth peels and reported as Peak Peel Force, with the respective second peel, third peel, fourth peel, and fifth peel distinctions, to the nearest 0.1 N.

### Hinge/ permanent seal strength

The seal strength of the permanent seal portion (i.e., hinge area) of the reclosable panel on an absorbent article package is determined by measuring the force required to separate one side of the seal (e.g., the second sheet) from the opposing side of the seal (e.g. the first sheet) by executing a typical peel test using a universal constant rate of extension test frame. All measurements are performed in a laboratory maintained at 23 °C ± 2 C° and 50% ± 2% relative humidity and test samples are conditioned in this environment for at least 2 hours prior to testing.

A suitable universal constant rate of extension (CRE) test frame is the MTS Alliance interfaced to a computer running TestSuite control software (available from MTS Systems Corp, Eden Prairie, MN), or equivalent. The CRE test frame is equipped with a load cell for which forces measured are within 1% to 99% of the limit of the cell. The fixtures used to grip the test specimen are clamps with knife or serrated edge grip faces that have a width equal to or greater than the width of the upper and lower legs of the prepared permanent seal test sample. The fixtures are installed on the CRE test frame and mounted such that they are horizontally and vertically aligned with one another.

A test sample of the permanent seal portion of a reclosable panel on an absorbent article package is prepared as follows. First, the entire reclosable panel that includes the reclosable seals, the hinge, and the permanent seal is excised from the package in a manner that prevents any disturbance or pre-peeling of any of the seals present. Now slowly peel open only the reclosable portion of the reclosable panel by separating the second sheet from the underlying first sheet, stopping the peel when the first edge of the permanent seal begins, thereby creating an upper leg (i.e., second sheet material) and a lower leg (i.e., first sheet material) adjacent to the permanent seal. In like fashion, a total of five replicate permanent seal test samples from the reclosable panel of five separate absorbent article packages are prepared.

Program the CRE test frame for a constant rate of extension uniaxial elongation with a set path length as follows. The gauge length is governed by the overall length of the prepared test sample (the sum of both legs) and is set to a distance that is about 20 mm less than the overall length of the prepared test sample. This gauge length allows the legs to be clamped in the grips of the CRE test frame. The path length is governed by the length of the permanent seal. Generally, the path length is set to a distance that is about 5 mm greater than the permanent seal length. The grips are programmed to move closer together (from the zeroed position) by an intentional slack of 1.0 mm to ensure no pretension force exists on the test sample at the onset of the test. The grips are then programmed to move apart at a slack speed of 1.0 mm/s for the initial slack distance of 1.0 mm, then continue to move apart at a speed of 5.0 mm/s until the opposing sides of the seal (i.e., first and second sheets of the permanent seal) are fully separated and the extension endpoint is reached. The grips are then returned to the zero position.

The peel test is executed as follows. The upper leg of the prepared test sample is clamped into the upper grip of the CRE test frame such that width of the leg is centered laterally within the grip. In like fashion, the lower leg of the prepared test sample is clamped into the lower grip of the CRE test frame. The position of the test sample is adjusted, as needed, so that the permanent seal is located approximately equidistant between the grips and the width of the permanent seal is perpendicular to the pull direction of the crosshead. The legs can be trimmed, if needed, and the distance between the grips can be adjusted, as needed, to minimize the slack but ensuring ≤ 0.1 N of tension is exerted to prevent pre-mature peeling of the permanent seal. The crosshead is then zeroed. The peel test is started and force ("load") and displacement data is continuously collected throughout the test at a data acquisition rate of 100 Hz. When the crosshead has returned to the zero position, the legs of the test sample are removed from the grips and the test sample is discarded.

Construct a graph of force (N) versus displacement (mm). From the graph, determine the peak force and record as peak peel force within the hinge to the nearest 0.1 N.

In like fashion, repeat the entire process until peel testing has been executed on all five replicate test samples of the permanent seal.

The arithmetic mean of the peak force among the five permanent seal test sample replicates is calculated and reported as Peak Peel Force within the hinge region to the nearest 0.1 N.

### Strength Tensile Test Method

The tensile properties (tensile strength, stretch and energy absorption) of a test sample are calculated from measured force and elongation values obtained using a constant rate of elongation test until the sample breaks. The test is run in accordance with compendia! method ISO 1924-3, with modifications noted herein. Measurements are made on a constant rate of extension tensile tester using a load cell for which the forces measured are within 1 % to 99% of the limit of the cell.

A suitable instrument is the MTS Alliance using Test Suite Software, available from MTS SystemsCorp., Eden Prairie, MN, or equivalent. All measurements are performed in a laboratory maintained at 23 °C ± 2 C0 and 50% ± 2% relative humidity and test samples are conditioned in this environment for at least 2 hours prior to testing.

Measurements are made on both MD (machine direction) and CD (cross direction) test samples taken from rolls or sheets of the raw material, or test samples obtained from a finished package. When excising the test sample from a finished package, use care to not impart any contamination or distortion to the sample during the process. The excised sample should be free from residual adhesive and taken from an area of the package that is free from any seams or folds. The test sample is cut to a width of 25.4 mm with a length that can accommodate a test span of 50.8 mm. The long side of the sample is parallel to the direction of interest (MD, CD). Normally in finished packages, the MD runs from the bottom to the top of the package, but this can be verified by determining the fiber orientation if in doubt. Ten replicate test samples should be prepared from the MD and ten additional replicates from the CD.

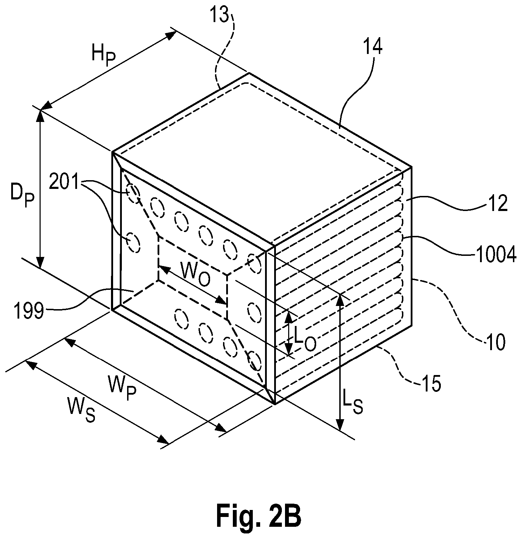

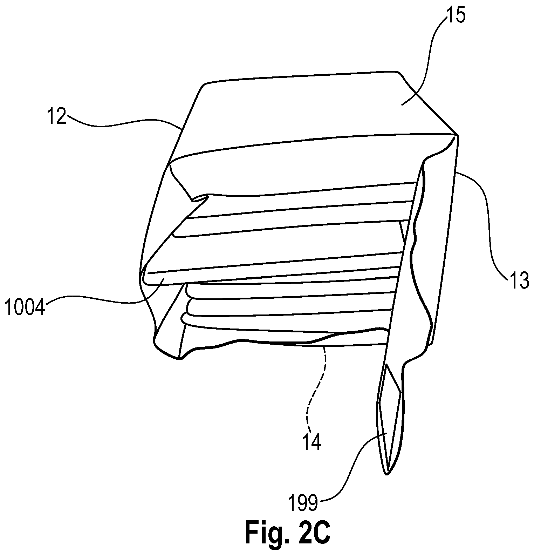

Program the tensile tester for a constant rate of extension uniaxial elongation to break as follows. Set the gauge length (test span) to 50.8 mm using a calibrated gauge block and zero the crosshead. Insert the test sample into the grips such that the long side is centered and parallel to the central pull axis of the tensile tester. Raise the crosshead at a rate of 25.4 mm/min until the test sample breaks, collecting force (N) and extension (mm) data at 100 Hz throughout the test. Construct a graph of force (N) versus extension (mm). Read the maximum force (N) from the graph and record as Peak Force to the nearest 0. 1 N, noting MD or CD. Read the extension at the maximum force (N) from the graph and record as Elongation at Break to the nearest 0.01 mm, noting MD or CD. From the graph, determine the point (z) where the tangent to the curve, with a slope equal to the maximum slope of the curve, intersects the elongation axis. Now calculate the area under the force vs elongation curve from point z up to the point of maximum force and report to the nearest 0.1 mJ, noting MD or CD. [Refer to Figure 2 in ISO 1924-3 for a depiction of a typical force vs elongation curve where point z is denoted.]

Calculate the arithmetic mean Peak Force for all MD replicates and then all CD replicates and record respectively as Mean MD Peak Force and Mean CD Peak Force to the nearest 0.1 N. Calculate the arithmetic mean Elongation at Break for all MD replicates and then all CD replicates 30 and record respectively as Mean MD Elongation at Break and Mean CD Elongation at Break to the nearest 0.01 mm. Calculate the arithmetic mean area under the force vs elongation curve for all MD replicates and then all CD replicates and record respectively as Mean Area Under MD Curve and Mean Area Under CD Curve to the nearest 0. 1 mJ.

Tensile strength is calculated by dividing the Mean Peak Force (N) by the width of the test sample (25.4 mm). Calculate the tensile strength for the MD replicates and then the CD replicates and report respectively as MD Tensile Strength and CD Tensile Strength to the nearest 0. 1 kN/m.

Stretch at break is calculated by dividing the Mean Elongation at Break (mm) by the initial test length (test span) of 50.8 mm, and then multiplying by 100. Calculate the stretch at break for the MD replicates and then the CD replicates and report respectively as MD Stretch at Break and CD Stretch at Break to the nearest percent.

### Burst Strength Test Method

Burst strength is the maximum uniformly distributed pressure that a test sample can withstand. Burst strength is measured in accordance with compendial method ISO 2758:2014 using a test apparatus as described within the method. A suitable instrument is the 13-60 Burst Tester for Paper and Foils available from Testing Machines, Inc (New Castle, DE), or equivalent. The instrument is calibrated and operated as per the manufacturer's instructions. All measurements are performed in a laboratory maintained at 23°C +/- 2 C° and 50% +/- 2% relative humidity, and test samples are conditioned in this environment for at least 2 hours prior to testing.

Measurements are made on test samples taken from rolls or sheets of the raw material, or test specimens obtained from a finished package. When excising a test sample from a finished package, use care to not impart any contamination or distortion to the test sample during the process. The test sample must be larger than the clamps used to hold the test sample in the instrument. The test sample should be taken from an area free of folds, wrinkles, or seams.

Measure the burst strength (using a clamping pressure sufficient to prevent slippage during the test, and a pumping rate of 95 ± 15 mL/min) for a total of 10 replicate test samples. For samples that are sided, the side of the test sample that is meant to face the inside of the package faces the pressure when placed into the clamps, and 10 replicates are tested in this orientation. For samples that are balanced (not sided), 5 replicates are tested with the inside of the package facing the pressure and 5 replicates are tested with the outside of the package facing the pressure, and the results are averaged together. Record the pressure at which each test sample bursts to the nearest 0.001 kPa. If the burst pressure is less than 70 kPa, multiple layers of the test material must be used. To obtain the burst strength, divide the burst pressure by the number of layers tested. Calculate the arithmetic mean burst pressure for all replicates and report as Burst Strength to the nearest 0.001 kPa.

### Caliper Test Method

The caliper, or thickness, of a single-layer test sample is measured under a static load by a micrometer, in accordance with compendial method ISO 534:2001, with modifications noted herein. All measurements are performed in a laboratory maintained at 23 °C ± 2 C° and 50% ± 2% relative humidity and test samples are conditioned in this environment for at least 2 hours prior to testing.

Caliper is measured with a micrometer equipped with a pressure foot capable of exerting a steady pressure of 70 kPa ± 0.05 kPa onto the test sample. The micrometer is a dead-weight type instrument with readings accurate to 0.1 micron. A suitable instrument is the TMI Digital Micrometer Model 49-56, available from Testing Machines Inc., New Castle, DE, or equivalent. The pressure foot is a flat ground circular movable face with a diameter that is smaller than the test specimen and capable of exerting the required pressure. A suitable pressure foot has a diameter of 16.0 mm. The test sample is supported by a horizontal flat reference platform that is larger than and parallel to the surface of the pressure foot. The system is calibrated and operated per the manufacturer's instructions.

Measurements are made on single-layer test samples taken from rolls or sheets of the raw material, or test samples obtained from a finished package. When excising the test sample from a finished package, use care to not impart any contamination or distortion to the sample during the process. The excised sample should be free from residual adhesive and taken from an area of the package that is free from any seams or folds. The test sample is ideally 200 mm² and must be larger than the pressure foot.

To measure caliper, first zero the micrometer against the horizontal flat reference platform. Place the test sample on the platform with the test location centered below the pressure foot. Gently lower the pressure foot with a descent rate of 3.0 mm per second until the full pressure is exerted onto the test sample. Wait 5 seconds and then record the caliper of the test sample to the nearest 0.1 micron. In like fashion, repeat for a total of ten replicate test samples. Calculate the arithmetic mean for all caliper measurements and report the value as Caliper to the nearest 0.1 micron.

### Basis Weight Test Method

The basis weight of a test sample is the mass (in grams) per unit area (in square meters) of a single layer of material and is measured in accordance with compendial method ISO 536:2019. The mass of the test sample is cut to a known area, and the mass of the sample is determined using an analytical balance accurate to 0.0001 grams. All measurements are performed in a laboratory maintained at 23 °C ± 2 C° and 50% ± 2% relative humidity and test samples are conditioned in this environment for at least 2 hours prior to testing.

Measurements are made on test samples taken from rolls or sheets of the raw material, or test samples obtained from a finished package. When excising the test sample from a finished package, use care to not impart any contamination or distortion to the sample during the process. The excised sample should be free from residual adhesive and taken from an area of the package that is free from any seams or folds. The test sample must be as large as possible so that any inherent material variability is accounted for.

Measure the dimensions of the single layer test sample using a calibrated steel metal ruler traceable to NIST, or equivalent. Calculate the Area of the test sample and record to the nearest 0.0001 square meter. Use an analytical balance to obtain the Mass of the test sample and record to the nearest 0.0001 gram. Calculate Basis Weight by dividing Mass (in grams) by Area (in square meters) and record to the nearest 0.01 grams per square meter (gsm). In like fashion, repeat for a total of ten replicate test samples. Calculate the arithmetic mean for Basis Weight and report to the nearest 0.01 grams/square meter.

### In-Bag Stack Height Test Method

The in-bag stack height of a package of absorbent articles is determined as follows:

### Equipment

A thickness tester with a flat, rigid horizontal sliding plate is used. The thickness tester is configured so that the horizontal sliding plate moves freely in a vertical direction with the horizontal sliding plate always maintained in a horizontal orientation directly above a flat, rigid horizontal base plate. The thickness tester includes a suitable device for measuring the gap between the horizontal sliding plate and the horizontal base plate to within ± 0.5 mm. The horizontal sliding plate and the horizontal base plate are larger than the surface of the absorbent article package that contacts each plate, i.e., each plate extends past the contact surface of the absorbent article package in all directions. The horizontal sliding plate exerts a downward force of 850 ± 1 gram-force (8.34 N) on the absorbent article package, which may be achieved by placing a suitable weight on the center of the non-package-contacting top surface of the horizontal sliding plate so that the total mass of the sliding plate plus added weight is 850 ± 1grams.

### Test Procedure

Absorbent article packages are equilibrated at 23 ± 2 °C and 50 ± 5 % relative humidity prior to measurement.

The horizontal sliding plate is raised, and an absorbent article package is placed centrally under the horizontal sliding plate in such a way that the absorbent articles within the package are in a horizontal orientation (see Fig. 6). Any handle or other packaging feature on the surfaces of the package that would contact either of the plates is folded flat against the surface of the package so as to minimize their impact on the measurement. The horizontal sliding plate is lowered slowly until it contacts the top surface of the package and then released. The gap between the horizontal plates is measured to within ± 0.5 mm ten seconds after releasing the horizontal sliding plate. Five identical packages (same size packages and same absorbent articles counts) are measured, and the arithmetic mean is reported as the package width. The "In-Bag Stack Height" = (package width/absorbent article count per stack) × 10 is calculated and reported to within ± 0.5 mm.

### Contemplated Examples

A1. A cuboidal package (1) comprising one or more absorbent articles (1004) comprising:
   a first sheet (99) of a first package material comprising natural fibers, which forms a front panel (14), a back panel (15) opposite the front panel (14), a first side panel (12), and a second side panel (13) opposite the first side panel (12);
   a top panel (11) and a bottom panel (10) opposite the top panel (11),
   wherein the panels define an interior compartment of the package (1), and wherein one or more absorbent articles are enclosed in the interior compartment;
   wherein the bottom panel (10) is at least partially formed by the first sheet (99);
   wherein the top panel (11) is partially formed by the first sheet (99);
   the package (1) further comprising a second sheet of a second package material partially forming the top panel (11),
   wherein the second sheet and the first sheet (99) form the top panel (11);
   wherein the second sheet is attached to the first sheet (99) such that a hinge is formed,
      wherein the top panel (11) comprises an opening area (199); and
      wherein the top panel (11) is at least partially reclosable via the second sheet.
A2. The package of example A1, wherein in the opening area (199), the second sheet is not attached to the first sheet (99) or is attached to the first sheet (99) such that the opening area (199) exhibits a Peak Peel Force at first peel of from 0.0 N to 10 N, preferably from 0.0 N to 5.0 N.
A3. The package of any one of examples A1-A2, wherein the opening area (199) comprises a corner of the second sheet, a perimeter position of the second sheet, or combinations thereof.
A4. The package of any one of examples A1-A3, wherein the second sheet is attached to the first sheet (99) via one or more adhesives.
A5. The package of any one of examples A1-A4, wherein the opening area (199) is either free of adhesives or the adhesive in the opening area (199) is covered by a coating or cover material.
A6. The package of any one of examples A1-A5, wherein an area, preferably an area comprising at least one corner or at least partially at least one side edge, of the second sheet is permanently attached to the first sheet (99) to form a hinge area.
A7. The package of example A6, wherein the hinge area exhibits Peak Peel Force of at least 10 N, preferably at least 20 N according to the Seal Strength Test Method disclosed herein.
A8. The package of any one of examples A1-A7, wherein an area, preferably an area comprising at least partially at least one side edge or at least one corner, of the second sheet is reclosably attached to the first sheet (99) to form a reclose area.
A9. The package of claim A8, wherein in the reclose area,
   the surface of the second sheet facing the first sheet comprises an adhesion-reducing coating and the surface of the first sheet (99) facing the second sheet comprises an adhesive; or
   the surface of the second sheet facing the first sheet comprises an adhesive and the surface of the first sheet (99) facing the second sheet comprises an adhesion-reducing coating.
A10. The package of examples A8 or A9, wherein the reclose area exhibits a Peak Peel Force at first peel of from 0.1 N to 20 N, preferably from 3.0 N to 15 N according to the Seal Strength Test Method disclosed herein.
A11. The package of any one of examples A8 to A10, wherein the opening area (199) exhibits a Peak Peel Force at first peel which is less than the Peak Peel Force at first peel of the reclose area according to the Seal Strength Test Method disclosed herein.
A12. The package of any one of examples A6 to A11, wherein the hinge area and the opening area (199) are located on opposing side edge or on opposing corners of the second sheet.
A13. The package of any one of examples A8 to A12, wherein the hinge area and the opening area (199) each comprise one corner of the second sheet, while the reclose area comprises the other two corners of the second sheet.
A14. The package of any one of examples A1-A13, wherein the first sheet (99) is envelope folded to partially form the top panel (11) and/ or at least partially form the bottom panel (10).
A15. The package of any one of any one of examples A1-A14, wherein the second package material comprises natural fibers.
A16. The package of any one of any one of examples A1-A15, wherein the package (1) further comprises a third sheet of a third package material comprising natural fibers and partially forming the bottom panel (10), wherein the third sheet is at attached to the part of the bottom panel (10) formed by the first sheet (99) such that the bottom panel (10) is formed.
A17. The package of any one of examples A1-A16, wherein the first and/ or second and/ or third package material has a Basis Weight between about 50 gsm and about 120 gsm, preferably between about 55 gsm and about 115 gsm, more preferably between about 60 gsm and about 110 gsm, according to the Basis Weight Test Method disclosed herein.
A18. The package of any one of examples A1-A17, wherein the second package material has a higher Basis Weight than the first package material according to the Basis Weight Test Method disclosed herein.
A19. The package of any one of examples A1-A17, wherein the first package material, the second package material and/or the third package material are the same package material.
A20. The package of any one of examples A1-A19, wherein the part of the top panel (11) formed by the first sheet (99) defines an opening (71), which is covered by the second sheet, having a length L_{O} and a width W_{O}.
A21. The package of example A20, wherein a surface area of the second sheet covering the opening (71) and facing the enclosed absorbent articles (1004) is free of adhesive.
A22. The package of example A20 or A21, wherein L_{O} is less than the dimension of the enclosed absorbent articles in the direction parallel to L_{O} and/ or W_{O} is less than the dimension of the one or more enclosed absorbent articles in the direction parallel to W_{O}.
A23. The package of any one of examples A1-A22, wherein the part of the top panel (11) formed by the first sheet (99) is partially folded over itself to form a folded area and wherein at least a portion of the folded area is not adhesively attached to itself.
A24. The package of example A23, wherein the second sheet of the second packaging material at least partly covers the folded area and wherein the folded area is able to unfold upon removal of the second sheet from the folded area.
A25. The package of examples A22 to A24, wherein, upon at least partial unfolding of the folded area, the opening (71) is expanded such that the absorbent articles fit through the expanded opening.
A26. The package of any one of examples A1-A25, wherein each absorbent article is folded at least once and are arranged within the package such that a fold nose faces the top panel (11).
A27. The package of any one of examples A1-A26, wherein the package comprises an opening indicium, preferably comprised by an outer surface of the reclose area.
A28. The package of any one of examples A1-A27, wherein the package exhibits a recyclable percentage of between about 60 percent to about 99.9 percent determined via test PTS-RH:021/97 (Draft Oct. 2019) under category II, as performed by Papiertechnische Stiftung located at Pirnaer Strasse 37, 01809 Heidenau, Germany.
A29. The package of example A28, wherein the one or more enclosed absorbent articles (1004) comprise between 10% and 95% by weight of the one or more enclosed absorbent articles of recycled material.
A30. The package of any one of the examples A1-A29, wherein the first and/ or second and/ or third package material comprises a barrier film layer disposed on an inner surface of the package material.
A31. The package of any one of the examples A1-A30, wherein the first and/ or second and/ or third package material comprises a barrier layer comprising metalized layer disposed on an inner surface of the package material.
A32. The package of example A31, wherein the first and/ or second and/ or third package material comprises a metalized layer comprising an Aluminiumoxide barrier or an Aluminiumoxide layer.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

Every document cited herein, including any cross referenced or related patent or application and any patent application or patent to which this application claims priority or benefit thereof, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests, or discloses any such invention. Further, to the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

## Claims

1. A cuboidal package (1) comprising one or more absorbent articles (1004) comprising:
a first sheet (99) of a first package material comprising natural fibers, which forms a front panel (14), a back panel (15) opposite the front panel (14), a first side panel (12), and a second side panel (13) opposite the first side panel (12);
a top panel (11) and a bottom panel (10) opposite the top panel (11),
wherein the panels define an interior compartment of the package (1), and wherein one or more absorbent articles are enclosed in the interior compartment;
wherein the bottom panel (10) is at least partially formed by the first sheet (99);
wherein the top panel (11) is partially formed by the first sheet (99);
the package (1) further comprising a second sheet of a second package material partially forming the top panel (11),
wherein the second sheet and the first sheet (99) form the top panel (11);
wherein the second sheet is attached to the first sheet (99) such that a hinge is formed,
wherein the top panel (11) comprises an opening area (199); and
wherein the top panel (11) is at least partially reclosable via the second sheet.

2. The package of claim 1, wherein in the opening area (199), the second sheet is not attached to the first sheet (99) or is attached to the first sheet (99) such that the opening area (199) exhibits a Peak Peel Force at first peel of from 0.0 N to 10 N, preferably from 0.0 N to 5.0 N.

3. The package of any one of the preceding claims, wherein the opening area (199) comprises a corner of the second sheet, a perimeter position of the second sheet, or combinations thereof.

4. The package of any one of the preceding claims, wherein the second sheet is attached to the first sheet (99) via one or more adhesives.

5. The package of any one of the preceding claims, wherein the opening area (199) is either free of adhesives or the adhesive in the opening area (199) is covered by a coating or cover material.

6. The package of any one of the preceding claims, wherein an area, preferably an area comprising at least one corner or at least partially at least one side edge, of the second sheet is permanently attached to the first sheet (99) to form a hinge area.

7. The package of claim 6, wherein the hinge area exhibits Peak Peel Force of at least 10 N, preferably at least 20 N according to the Seal Strength Test Method disclosed herein.

8. The package of any one of the preceding claims, wherein an area, preferably an area comprising at least partially at least one side edge or at least one corner, of the second sheet is reclosably attached to the first sheet (99) to form a reclose area.

9. The package of claim 8, wherein in the reclose area,
the surface of the second sheet facing the first sheet comprises an adhesion-reducing coating and the surface of the first sheet (99) facing the second sheet comprises an adhesive; or
the surface of the second sheet facing the first sheet comprises an adhesive and the surface of the first sheet (99) facing the second sheet comprises an adhesion-reducing coating.

10. The package of claims 8 or 9, wherein the reclose area exhibits a Peak Peel Force at first peel of from 0.1 N to 20 N, preferably from 3.0 N to 15 N according to the Seal Strength Test Method disclosed herein.

11. The package of any one of claims 8 to 10, wherein the opening area (199) exhibits a Peak Peel Force at first peel which is less than the Peak Peel Force at first peel of the reclose area according to the Seal Strength Test Method disclosed herein.

12. The package of any one of claims 6 to 11, wherein the hinge area and the opening area (199) are located on opposing side edge or on opposing corners of the second sheet.

13. The package of any one of claims 8 to 12, wherein the hinge area and the opening area (199) each comprise one corner of the second sheet, while the reclose area comprises the other two corners of the second sheet.

14. The package of any one of the preceding claims, wherein the first sheet (99) is envelope folded to partially form the top panel (11) and/ or at least partially form the bottom panel (10).

15. The package of any one of the preceding claims, wherein the second package material comprises natural fibers.
